(19) **Europäisches Patentamt**
**European Patent Office**
**Office européen des brevets**

(11) **EP 1 621 131 B1**

(12) **EUROPEAN PATENT SPECIFICATION**

(45) Date of publication and mention of the grant of the patent:
**25.07.2007 Bulletin 2007/30**

(51) Int Cl.:
*A61B 5/107* *(2006.01)* *A61B 5/053* *(2006.01)*

(21) Application number: **05016233.8**

(22) Date of filing: **26.07.2005**

(54) **Body composition meter**

Gerät zur Messung der Körperzusammensetzung

Dispositif de mesure de la composition corporelle

(84) Designated Contracting States:
**DE FR GB**

(30) Priority: **27.07.2004 JP 2004218697**
**06.06.2005 JP 2005166241**

(43) Date of publication of application:
**01.02.2006 Bulletin 2006/05**

(73) Proprietor: **TANITA CORPORATION**
**Tokyo (JP)**

(72) Inventor: **Kasahara, Yasuhiro**
**Itabashi-Ku**
**Tokyo (JP)**

(74) Representative: **Grünecker, Kinkeldey,**
**Stockmair & Schwanhäusser**
**Anwaltssozietät**
**Maximilianstrasse 58**
**80538 München (DE)**

(56) References cited:
**EP-A- 1 121 898** **US-A1- 2004 077 969**

• **PATENT ABSTRACTS OF JAPAN vol. 2003, no. 12, 5 December 2003 (2003-12-05) & JP 2004 135698 A (OMRON HEALTHCARE CO LTD), 13 May 2004 (2004-05-13)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

**Description**

BACKGROUND OF THE INVENTION

(i) Field of the Invention

This invention relates to a body composition meter capable of measuring impedances in a specific body part and estimating body composition indicators.

(ii) Description of the Related Art

In this field, body fat meters capable of measuring an impedance which occurs between both feet, both hands or a hand and a foot and estimating the body fat percentage of a whole body which is a body composition indicator are introduced to the market from the viewpoint of a health-oriented life, and along with an increase in desire for the health-oriented life, further research and development have been made, and body fat meters capable of measuring an impedance in the abdomen and estimating a subcutaneous fat amount and a visceral fat amount in the abdomen which are body composition indicators are disclosed.

Body fat meters disclosed in patent publications, i.e., those capable of estimating a subcutaneous fat amount and a visceral fat amount in the abdomen, attach current electrodes and measuring electrodes on a subject, pass a current between the current electrodes, and measure a voltage generated in the abdomen between the measuring electrodes when the current is passed between the current electrodes. All of these body fat meters are easy to use and capable of highly accurate measurements and estimations.

Patent Publication 1

Japanese Patent Laid-Open Publication No. 2001-178697 Patent Publication 2
Japanese Patent Laid-Open Publication No. 2001-252256 Patent Publication 3
Japanese Patent Laid-Open Publication No. 2001-252257 Patent Publication 4
Japanese Patent Laid-Open Publication No. 2002-238871 Patent Publication 5
Japanese Patent Laid-Open Publication No. 2002-369806 Patent Publication 6
Japanese Patent Laid-Open Publication No. 2004-135698 Patent Publication 7
Japanese Patent Laid-Open Publication No. 2004-141186

US 2004/077969 discloses a method of measuring a body fat quantity of an abdominal area of a subject by applying electric currents to a portion of the abdominal surface between one of the electrodes of a first electrode group which is attached to the abdominal surface of the body and one of the electrodes of a second electrode group which is attached to the back surface of the body measuring a voltage between two electrodes of the electrodes of a third electrode group which is attached to the surface of the body at an intermediate position between the first and the second electrode groups; and calculating the body FAT quantity of the abdominal area of the subject based on the measured voltage.

EP 121898 discloses an apparatus utilizing a one-chip microcomputer, in which all the functions necessary for the measurement of bioelectrical impedance are integrated into a circuit on the one-chip, and the apparatus enables uses to measure the bioelectrical impedance at a plurality of frequencies by utilizing a plurality of electrodes.

However, despite the above background, body fat meters which are easier to use and capable of more accurate measurements and estimations are demanded in the market.

Technical key points in improving the ease of use and accuracy of the body fat meters which measure an impedance in the abdomen and estimate a subcutaneous fat amount and a visceral fat amount in the abdomen are relative locations of the current electrodes and the measuring electrodes and a detection method therefor.

Thus, under the above circumstances, an object of the present invention is to provide a body composition meter which differs from the body fat meters disclosed in the patent publications in relative locations of current electrodes and measuring electrodes and detection method therefor and is capable of measuring impedances in a specific body part easily and estimating body composition indicators with high accuracy.

SUMMARY OF THE INVENTION

A body composition meter of the present invention is defined in present claim 1 and comprises multiple pairs of current electrodes,

a pair of measuring electrodes,
a current generating section,
a voltage detecting section, and
body composition estimating means,
wherein
the multiple pairs of current electrodes are disposed on a specific body part sequentially at smaller spacings,
the measuring electrodes are disposed between the innermost pair of current electrodes,
the current generating section generates a current between each pair of current electrodes,
the voltage detecting section detects a voltage generated between the measuring electrodes when the current generating section generates a current between each pair of current electrodes, and
the body composition estimating means estimates body composition indicators based on all of the voltages detected by the voltage detecting section.

Further, the body composition estimating means preferably comprises:

an impedance computing section,
a body composition computing equation storage section, and
a body composition computing section,
wherein
the impedance computing section computes an impedance based on a current generated between each pair of current electrodes by the current generating section and a voltage detected by the voltage detecting section,
the body composition computing equation storage section stores body composition computing equations for computing body composition indicators based on all of the impedances computed by the impedance computing section, and
the body composition computing section computes body composition indicators by substituting the impedances computed by the impedance computing section into the body composition computing equations stored in the body composition computing equation storage section.

Further, the body composition meter of the invention further comprises:

a body part width acquiring section, and
a display section,
wherein
the body part width acquiring section acquires the width of the specific body part, and
the display section displays the specific body part width acquired by the body part width acquiring section as the width of the shape of a displayed cross section of the specific body part and displays the body composition indicators estimated by the body composition estimating means as the size of the inside of the displayed cross section of the specific body part at the magnification used to display the width of the specific body part.

Further, the body composition meter of the invention further comprises a support that supports the multiple pairs of current electrodes and the measuring electrodes to make the electrodes contact the specific body part.
Further, the support has preferably a shape which fits the shape of the surface of the specific body part that makes contact with the electrodes and has a flexible portion at least in a portion thereof.
Preferably the support comprises:

a body part width acquiring section, and
a display section,
wherein
the body part width acquiring section measures the width of the specific body part, and
the display section displays the specific body part width measured by the body part width acquiring section as the width of the shape of a displayed cross section of the specific body part and displays the body composition indicators estimated by the body composition estimating means as the size of the inside of the displayed cross section of the specific body part at the magnification used to display the width of the specific body part.

Preferably, the body part width acquiring section comprises:

an adjustable arm, and
encoders,

wherein
the adjustable arm expands or contracts in the width direction of the specific body part to fit around the specific body part, and
the encoders detect the distance when the adjustable arm is fitted around the specific body part.

Preferably, the body part width acquiring section further comprises:

contact detecting sensors, and
a driving motor,
wherein
the contact detecting sensors detect that the adjustable arm is fitted around the specific body part, and
the driving motor expands or contracts the adjustable arm and stops the adjustable arm at positions where the contact detecting sensors detect that the adjustable arm is fitted around the specific body part.

Preferably, the specific body part is the abdomen of a body, and the body composition indicator is at least one selected from the group consisting of a subcutaneous fat thickness, an abdominal muscle thickness, a subcutaneous fat area, a visceral fat area, a total abdominal fat area, a truncal fat percentage and a whole body fat percentage.
Preferably, the distance between the multiple pairs of current electrodes and the measuring electrodes is a specific distance ranging from 4 cm to 10 cm.
Preferably, the specific distance between the outermost pair of current electrodes and the measuring electrodes is 8 cm, and the specific distance between the innermost pair of current electrodes and the measuring electrodes is 4 cm to 5 cm.
Preferably, the distance between the measuring electrodes is 8 cm.
Preferably, the support changes the distances between ones of the current electrodes and the others of the current electrodes and the distance between one of the measuring electrodes and the other of the measuring electrodes according to the width of the specific body part.
Preferably, the body composition meter further comprises a body part width acquiring section which acquires the width of the specific body part, and

the body composition estimating means comprises:

an impedance computing section,
a body composition computing equation storage section, and
a body composition computing section,
wherein
the impedance computing section computes an impedance based on a current generated between each pair of current electrodes by the current generating section and a voltage detected by the voltage detecting section,
the body composition computing equation storage section stores body composition computing equations for computing body composition indicators based on the width of the specific body part acquired by the body part width acquiring section and all of the impedances computed by the impedance computing section, and
the body composition computing section computes body composition indicators by substituting the impedances computed by the impedance computing section into the body composition computing equations stored in the body composition computing equation storage section.

Preferably, the current generating section generates a current such that the frequency of the current gradually lowers from the outermost pair of current electrodes to the innermost pair of current electrodes.
Preferably, the current generating section generates a current of specific frequency ranging from 128 kHz to 512 kHz between the outermost pair of current electrodes and generates a current of specific frequency ranging from 4 kHz to 12.5 kHz between the innermost pair of current electrodes.
Preferably, the specific frequency ranging from 128 kHz to 512 kHz is a frequency of 256 kHz, and the specific frequency ranging from 4 kHz to 12.5 kHz is a frequency of 5 kHz.
The body composition meter of the present invention has the multiple pairs of current electrodes placed on a specific body part successively at smaller spacings, generates a current between each pair of current electrodes in the current generating section, detects a voltage generated between the measuring electrodes when the current generating section generates a current between each pair of current electrodes in the voltage detecting section, and estimates body composition indicators based on all of these detected voltages in the body composition estimating means. Thus, by use of the above body composition meter, a user can estimate highly accurate body composition indicators taking into consideration the degree of current dependence on layers which are relatively shallow, relatively medium and relatively deep from the surface of a specific body part on which the electrodes are disposed, as easily as the user places the electrodes on the specific body part.

Preferably, the body composition estimating means estimates body composition indicators merely by substituting impedances computed by the impedance computing section into the body composition computing equations stored in the body composition computing equation storage section to compute the body composition indicators in the body composition computing section. Thereby, a user can estimate body composition indicators more easily.

Preferably, the display section displays the width of the shape of a displayed cross section of the specific body part and the size of body composition indicators of the inside of the displayed cross section at the same magnification as the actual width of a cross section of the specific body part and the actual size of body composition indicators of the inside of the cross section. Thereby, a user can know the accurate condition of a cross section of the specific body part easily.

Preferably, the multiple pairs of current electrodes and the measuring electrodes are disposed on the support. Thereby, the electrodes can be contacted with the specific body part easily.

Preferably, the support has a shape which fits the shape of the surface of the specific body part that contacts the electrodes and has a flexible portion at least in a portion thereof. Thereby, the support can be contacted with the specific body part accurately.

Preferably, the support also serves as the body part width acquiring section, and the display section displays the width of the shape of a displayed cross section of the specific body part and the size of body composition indicators of the inside of the displayed cross section at the same magnification as the actual width of a cross section of the specific body part and the actual size of body composition indicators of the inside of the cross section. Thereby, a user can know the accurate condition of a cross section of the specific body part more easily.

Preferably, in the body part width acquiring section, the adjustable arm is fitted around the specific body part, and the encoders detect the distance when the adjustable arm is fitted around the specific body part. Thereby, a user can acquire the width of the body part easily even in a lying position.

Preferably, in the body part width acquiring section, the driving motor extends or contracts the adjustable arm automatically and stops the movement of the adjustable arm at a position where the contact detecting sensors have detected that the adjustable arm is fitted around the specific body part. Thus, a user can acquire the width of the body part more easily.

Preferably, the specific body part is the abdomen of a body, and the body composition indicator is at least one selected from the group consisting of a subcutaneous fat thickness, an abdominal muscle thickness, a subcutaneous fat area, a visceral fat area, a total abdominal fat area, a truncal fat percentage and a whole body fat percentage. Thus, a user can easily estimate at least one selected from the group consisting of a subcutaneous fat thickness, an abdominal muscle thickness, a subcutaneous fat area, a visceral fat area, a total abdominal fat area, a truncal fat percentage and a whole body fat percentage from the abdomen of a body.

Preferably, the distance between the multiple pairs of current electrodes and the measuring electrodes is a specific distance ranging from 4 cm to 10 cm. Thereby, a current can be passed from the current generating section with good dependency on the layers from the surface of the specific body part, and estimation accuracy can be increased.

Preferably, the specific distance between the outermost pair of current electrodes and the measuring electrodes is 8 cm, and the specific distance between the innermost pair of current electrodes and the measuring electrodes is 4 cm to 5 cm. Thereby, a current can be passed from the current generating section with better dependency on the layers from the surface of the specific body part with higher dependency, and estimation accuracy can be further increased.

Preferably, the distance between the measuring electrodes is 8 cm. Thus, a voltage corresponding to a current passing depending on the layers from the surface of the specific body part can be detected in the voltage detecting section with high accuracy, and estimation accuracy can be further increased.

Preferably, the support changes the distances between ones of the current electrodes and the others of the current electrodes and the distance between one of the measuring electrodes and the other of the measuring electrodes according to the width of the specific body part. Thereby, regardless of the width of the specific body part, accurate impedances in the abdomen can be obtained, and body composition indicators can be estimated.

Preferably, the body composition estimating means estimates body composition indicators merely by substituting the width of the specific body part acquired by the body part width acquiring section and impedances computed by the impedance computing section into the body composition computing equations stored in the body composition computing equation storage section to compute the body composition indicators in the body composition computing section. Thereby, a user can estimate body composition indicators more accurately and more easily.

Preferably, the current generating section generates a current such that the frequency of the current gradually lowers from the outermost pair of current electrodes to the innermost pair of current electrodes. Thereby, voltages can be detected according to distribution of tissues in the specific body part in the voltage detecting section, and estimation accuracy can be increased.

Preferably the current generating section generates a current of specific frequency ranging from 128 kHz to 512 kHz between the outermost pair of current electrodes and a current of specific frequency ranging from 4 kHz to 12.5 kHz between the innermost pair of current electrodes. Thereby, voltages with a high degree of reflection can be detected according to distribution of tissues in the specific body part in the voltage detecting section, and estimation accuracy can be further increased.

Preferably the current generating section generates a current of 256 kHz between the outermost pair of current electrodes and a current of 5 kHz between the innermost pair of current electrodes. Thereby, voltages with a higher degree of reflection can be detected according to distribution of tissues in the specific body part in the voltage detecting section, and estimation accuracy can be further increased.

BRIEF DESCRIPTION OF THE DRAWINGS

Fig. 1 is diagrams illustrating an external structure of a body composition meter according to the present invention, wherein A is a plan view and B is a front view (Example 1).
Fig. 2 is a block diagram illustrating the constitution of the body composition meter according to the present invention (Examples 1 and 2).
Fig. 3 is a block diagram illustrating the operation procedures of the body composition meter according to the present invention (Examples 1 and 2).
Fig. 4 is a graph illustrating the correlation between a subcutaneous fat thickness measured by CT scanning and a subcutaneous fat thickness computed by a body composition computing equation (Example 1).
Fig. 5 is a graph illustrating the correlation between an abdominal muscle thickness measured by CT scanning and an abdominal muscle thickness computed by a body composition computing equation (Example 1).
Fig. 6 is a graph illustrating the correlation between a subcutaneous fat area measured by CT scanning and a subcutaneous fat area computed by a body composition computing equation (Example 1).
Fig. 7 is a graph illustrating the correlation between a visceral fat area measured by CT scanning and a visceral fat area computed by a body composition computing equation (Example 1).
Fig. 8 is a graph illustrating the correlation between a total abdominal fat area measured by CT scanning and a total abdominal fat area computed by a body composition computing equation (Example 1).
Fig. 9 is a graph illustrating the correlation between a truncal fat percentage measured by DXA and a truncal fat percentage computed by a body composition computing equation (Example 1).
Fig. 10 is a graph illustrating the correlation between a whole body fat percentage measured by DXA and a truncal fat percentage measured by DXA (Example 1).
Fig. 11 is a graph illustrating the correlation between a truncal fat percentage measured by DXA and the reciprocal of a measured abdominal impedance (Example 1).
Fig. 12 is diagrams illustrating examples of screens displayed in the display section, wherein A is a diagram illustrating an initial screen, B and C are diagrams illustrating screens displaying estimation results, and D is a diagram illustrating a screen for inquiring for remeasurement (Examples 1 and 2).
Fig. 13 is diagrams illustrating the principle of why body composition indicators can be determined, wherein A is a cross-sectional view of an abdominal portion which contacts the electrodes, B is a three dimensional view of the abdominal portion which contacts the electrodes, and C is a three dimensional view of an abdominal portion to be detected.
Fig. 14 is an electrical equivalent circuit model diagram of the abdomen of a body.
Fig. 15 is diagrams illustrating another external structure of the body composition meter according to the present invention, wherein A is a plan view and B is a front view.
Fig. 16 is diagrams illustrating another external structure of the body composition meter according to the present invention, wherein A is a plan view and B is a front view.
Fig. 17 is diagrams illustrating an external structure of a body composition meter according to the present invention, wherein A is a plan view and B is a front view (Example 2).
Fig. 18 is diagrams illustrating another external structure of the body composition meter according to the present invention, wherein A is a plan view and B is a front view.
Fig. 19 is diagrams illustrating another external structure of the body composition meter according to the present invention, wherein A is a plan view and B is a front view.

DETAILED DESCRIPTION OF THE PREFERRED EMBODIMENTS

The body composition meter of the present invention at least comprises multiple pairs of current electrodes, a pair of measuring electrodes, a current generating section, a voltage detecting section and body composition estimating means, measures impedances in a specific body part, and estimates indicators (such as an amount, thickness, area and percentage) associated with body composition (which is generally used as a generic term for body fat, visceral fat, subcutaneous fat, muscles, bones, body water and other body constituents).

The multiple pairs of current electrodes are disposed on a specific body part successively at smaller spacings. The measuring electrodes are disposed between the innermost pair of current electrodes. The current generating section generates a current between each pair of current electrodes out of the multiple pairs of current electrodes. The current generating section generates a current such that the frequency of the current gradually lowers from the outermost pair of current electrodes to the innermost pair of current electrodes. The voltage detecting section detects a voltage generated between the measuring electrodes when the current generating section generates a current between each pair of current electrodes. The body composition estimating means estimates body composition indicators based on all of the voltages detected by the voltage detecting section.

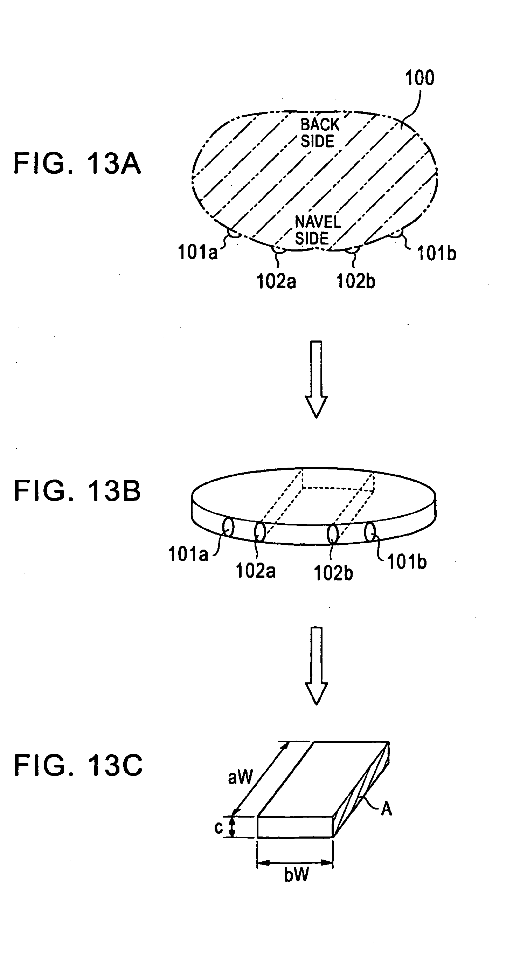

Next, the principle of why body composition indicators can be determined by measuring impedances in a specific body part will be described by use of Fig. 13. Fig. 13 is model diagrams for illustrating the principle when a pair of current electrodes and a pair of measuring electrodes are disposed on the navel side of the abdomen of a body. Fig. 13A is a cross-sectional view of the abdomen, Fig. 13B is a three dimensional view of a portion sliced out of the abdomen, and Fig. 13C is a three dimensional view of a sliced abdominal portion between the measuring electrodes.

It can be conceived that generating a current between a pair of current electrodes 101a and 101b to pass the current through an abdomen 100 and detecting a voltage between a pair of measuring electrodes 102a and 102b by the measuring electrodes 102a and 102b is equivalent to detecting a voltage when a current is passed through a conductor (object to be measured) which has a cross section A formed by the thickness aW in the anteroposterior direction (navel-back direction) of the abdomen 100 and the length c of the electrodes and a length bW between the measuring electrodes 102a and 102b. Further, it can be conceived that a body composition represents electrical resistivity since it reflects ease of passing of current.

Further, as is known, an impedance is represented by $Z = \rho L/S$ ($Z$: impedance, $\rho$: electrical resistivity, $L$: length, $S$: cross section). Thus, electrical resistivity $\rho$ for the specific area of the abdomen 100 of the body can be determined by substituting the cross section A formed by the thickness aW in the anteroposterior direction of the abdomen 100 and the length c of the electrodes into $S$, substituting the length bW between the measuring electrodes 102a and 102b into $L$, and substituting an impedance V/I based on a current $I$ generated between the current electrodes 101a and 101b and a voltage $V$ generated between the measuring electrodes 102a and 102b into $Z$. That is, measuring an impedance in a specific body part is equivalent to measuring an indicator associated with a body composition reflecting ease of passing of current in the specific body part.

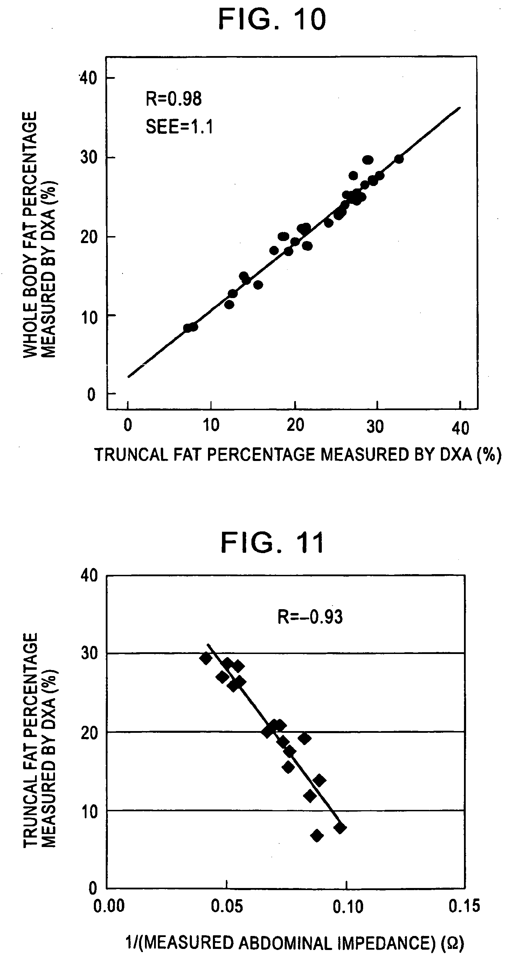

The validity of such a principle is also validated by a fact that the reciprocal of a measured abdominal impedance has a correlation with a truncal fat percentage which is a body composition indicator measured by DXA which is generally considered to have high estimation accuracy, as shown in the graph of Fig. 11 illustrating the correlation, for example.

Although a body composition indicator can be determined by such a principle, body composition distribution is not uniform from the surface of a specific body part toward the center of a body, and current density distribution from the surface of the specific body part toward the center of the body varies according to the distance between a pair of current electrodes disposed on the surface of the specific body part, when the specific body part is a cross section as shown in Fig. 13A. Thus, the accuracy of measurement of impedance may be low when a pair of current electrodes and a pair of measuring electrodes are disposed on the surface of a specific body part as shown in Fig. 13.

For example, when the distance between the current electrodes is short, the current density of a layer which is relatively shallow from the surface of the specific body part is high as compared with when the distance between the current electrodes is long. Further, the layer which is relatively shallow from the surface of the specific body part contains a large quantity of subcutaneous fat, and a layer which is relatively deep from the surface of the specific body part contains a large quantity of visceral fat. For this reason, an impedance based on measurement has a relatively high correlation with a body composition indicator when a subcutaneous fat area is estimated as the body composition indicator and has a relatively low correlation when a visceral fat area is estimated as the specific body part. Thus, deterioration of the accuracy of measurement of impedance in particular becomes significant depending on what is estimated as a body composition indicator.

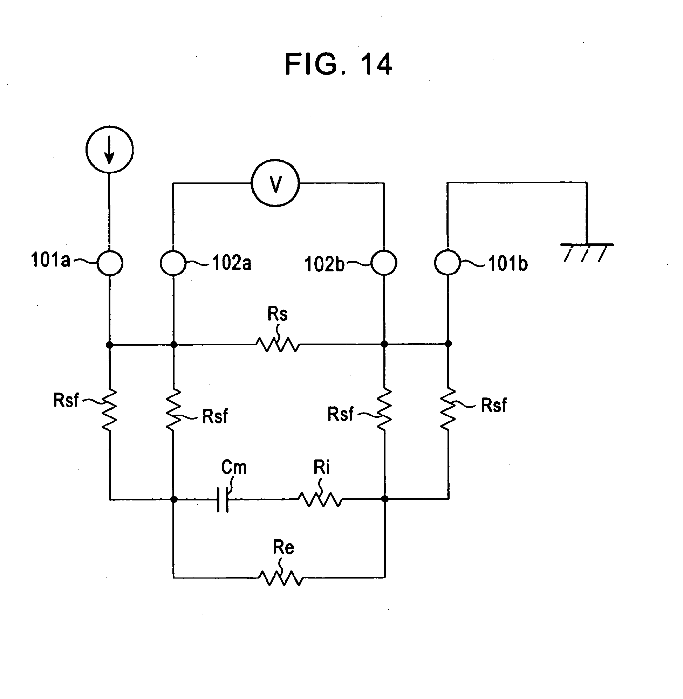

Further, a body composition depends on the frequency of a current passing through a specific body part. A description will be given to this point by use of Fig. 14. Fig. 14 is a diagram illustrating body tissues by an electrical equivalent circuit. As the body tissues, skin is represented by Rs, subcutaneous fat is represented by Rsf, a cell membrane is represented by Cm, an intracellular fluid is represented by Ri, and an extracellular fluid is represented by Re. Further, a muscle tissue is represented by the cell membrane Cm, the intracellular fluid Ri and the extracellular fluid Re. While a high-frequency current passes through all body tissues, a low-frequency current does not pass through the cell membrane Cm and the intracellular fluid Ri because the cell membrane Cm acts as an insulator. Thus, the accuracy of measurement of impedance is low due to the influence of the muscle tissue when a current of single frequency is passed.

For example, when a high-frequency current is passed through a specific body part, an impedance under the high influence of the muscle tissue is measured, and the correlation when a subcutaneous fat area is estimated as a body composition indicator is poor, while when a low-frequency current is passed through a specific body part, an impedance under the low influence of the muscle tissue is measured, and the correlation when a visceral fat area or a muscle thickness is estimated as a body composition indicator is poor.

The present body composition meter constituted as described above is based on the above principle and solves the problem of low accuracy when a pair of current electrodes and a pair of measuring electrodes are disposed.

To be more specific, according to the present body composition meter constituted as described above, since the multiple pairs of current electrodes are disposed on a specific body part successively at smaller spacings and a current is generated between each pair of current electrodes in the current generating section, a current passes such that it depends on a layer which is relatively shallow from the surface of the specific body part between an inner pair of current electrodes (distance between the current electrodes is short), a layer which is relatively deep from the surface of the specific body part between an outer pair of current electrodes (distance between the current electrodes is long), and a layer which is relatively medium from the surface of the specific body part between a pair of current electrodes (distance between the current electrodes is medium) disposed between the inner and outer pairs of current electrodes. Further, since a voltage generated between the measuring electrodes when the current generating section generates a current between each pair of current electrodes in the voltage detecting section and body composition indicators are estimated based on all of the detected voltages in the body composition estimating means, body composition indicators taking into consideration the degree of current dependence on the layers which are-relatively shallow, relatively medium and relatively deep from the surface of the specific body part can be estimated. Further, in particular, since the current generating section generates a current such that the frequency of the current gradually lowers from the outer pair of current electrodes to the inner pair of current electrodes, voltages can be detected according to distribution of tissues in the specific body part, and estimation accuracy can be further increased.

Therefore, the body composition meter of the present invention can obtain highly accurate body composition indicators without non-uniform body composition distribution from the surface of a specific body part toward the center of a body when a pair of current electrodes and a pair of measuring electrodes are disposed on the surface of the specific body part, varying current density distribution from the surface of the specific body part toward the center of the body according to the distance between the current electrodes disposed on the surface of the specific body part, and dependency of tissues on frequency.

Example 1

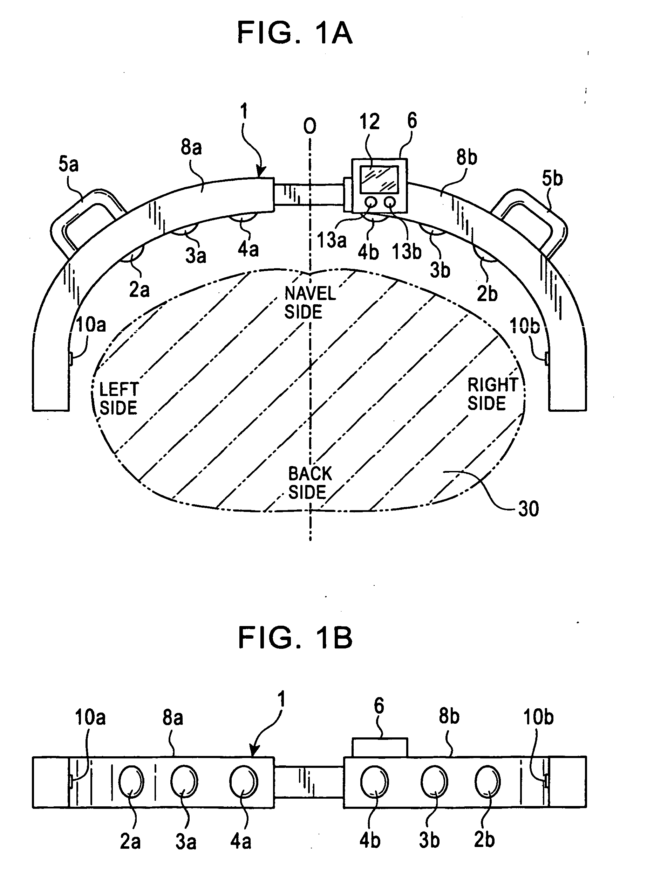

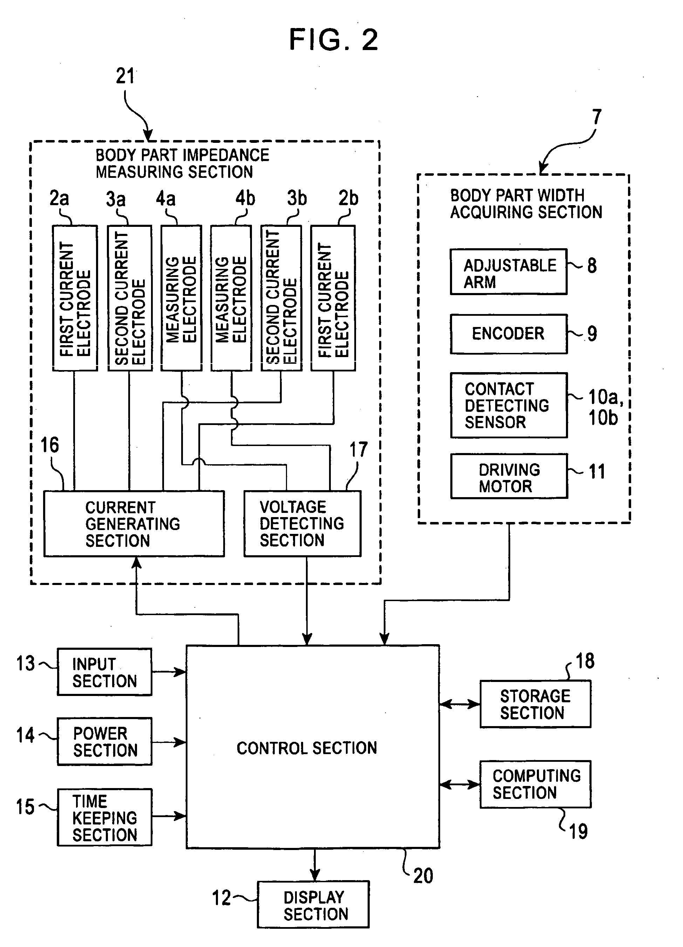

First, the specific constitution of a body composition meter (device which estimates body composition indicators in the abdomen by use of electrodes whose distance therebetween varies according to the width of the abdomen of a body) according to the present invention will be described by using primarily an external view shown in Fig. 1 and a block diagram shown in Fig. 2.

When viewed from outside, the body composition meter as Example 1 comprises a support 1 that comprises a group of electrodes (first current electrodes 2a and 2b, second current electrodes 3a and 3b, and measuring electrodes 4a and 4b), handles 5a and 5b, and an operation box 6.

The support 1 supports the electrodes, has a horseshoe shape so that the electrodes closely contact the abdomen 30 of a body, and also has a body part width acquiring section 7. The body part width acquiring section 7 measures the width of the abdomen of a body (i.e., the distance between the left and right sides of the abdomen) by expanding or contracting its central portion. More specifically, the support 1 comprises an adjustable arm 8 (i.e., two semi-horseshoe-shaped arms 8a and 8b). One end of the semi-horseshoe-shaped arm 8a slides into the inside of one end of the semi-horseshoe-shaped arm 8b and slides along the internal surface thereof. Further, the sliding ends of the two semi-horseshoe-shaped arms 8a and 8b have an encoder 9 which comprises electrodes for detecting capacitance. The encoders 9 detect the distance when the two semi-horseshoe-shaped arms 8a and 8b are fitted around the abdomen 30 of the body. Further, one end of the semi-horseshoe-shaped arm 8a forms a rack, one end of the semi-horseshoe-shaped arm 8b houses a driving motor 11 whose rotating shaft forms a pinion, and when the driving motor 11 drives to rotate the pinion-shaped rotating shaft with the rack-shaped portion engaging with the pinion-shaped portion, the rack-shaped portion moves. That is, the two semi-horseshoe-shaped arms 8a and 8b slide and extend or contract automatically. Further, contact detecting sensors 10a and 10b (such as photointerrupters and piezoelectric sensors) which detect that the semi-horseshoe-shaped arms 8a and 8b are in contact with the abdomen of the body are provided on the inner sides of the other ends of the two semi-horseshoe-shaped arms 8a and 8b.

The handles 5a and 5b are provided on the outer sides of the bended portions of the adjustable arm 8 to help a user to hold the support 1 easily.

The first current electrodes 2a and 2b are provided on the inner sides of the semi-horseshoe-shaped arms 8a and 8b, respectively, to pass a current through the abdomen 30 of the body. The second current electrodes 3a and 3b are provided on the inner sides of the semi-horseshoe-shaped arms 8a and 8b, respectively, and between the first current electrodes 2a and 2b to pass a current through the abdomen 30 of the body. The measuring electrodes 4a and 4b are provided on the inner sides of the semi-horseshoe-shaped arms 8a and 8b, respectively, and between the second current electrodes 3a and 3b to detect a voltage generated by passing a current through the abdomen 30 of the body. The electrodes 2a, 3a and 4a and 2b, 3b and 4b are placed symmetrically with respect to the O axis such that the distance between the measuring electrodes 4a and 4b is 8 cm, the distance between the second current electrodes 3a and 3b is 16 cm to 20 cm (preferably 16 cm to 18 cm), and the distance between the first current electrodes 2a and 2b is 24 cm to 28 cm (preferably 24 cm), when the arms 8a and 8b are in the most contracted state. In other words, the electrodes 2a, 3a and 4a and 2b, 3b and 4b are placed symmetrically with respect to the O axis such that the distance between the measuring electrode 4a and the second current electrode 3a and between the measuring electrode 4b and the second current electrode 3b is 4 cm to 6 cm (preferably 4 cm to 5 cm), and the distance between the measuring electrode 4a and the first current electrode 2a and between the measuring electrode 4b and the first current electrode 2b is 8 cm to 10 cm (preferably 8 cm). The term "distance" used herein refers to the distance from the edge of one electrode to the edge of the other electrode.

The operation box 6 has a display section 12 and an input section 13 on the front face of a case and incorporates a power section 14, a time keeping section 15, a current generating section 16, a voltage detecting section 17, a storage section 18, a computing section 19 and a control section 20.

The power section 14 supplies electric power to the sections constituting the electrical system of the present device.

The time keeping section 15 keeps time.

The input section 13 comprises a power switch 13a and a measurement switch 13b. The power switch 13a is used to start supplying electric power from the power section 14. The measurement switch 13b is used to start measurement of impedance.

The current generating section 16 switches and selects between connections of the electrodes for passing a current (i.e., between connection of the first current electrodes 2a and 2b and connection of the second current electrodes 3a and 3b) and generates a current to be passed through the abdomen 30 of the body under control of the control section 20. The current generating section 16 generates a high-frequency current Aout-high (having a frequency of 128 kHz to 512 kHz, preferably a frequency of 256 kHz) for the first current electrodes 2a and 2b and generates a low-frequency current Ain-low (having a frequency of 4 kHz to 12.5 kHz, preferably a frequency of 5 kHz) for the second current electrodes 3a and 3b.

The voltage detecting section 17 detects a voltage Vout-high generated between the measuring electrodes 4a and 4b when the current Aout-high is passed between the first current electrodes 2a and 2b and a voltage Vin-low generated between the measuring electrodes 4a and 4b when the current Ain-low is passed between the second current electrodes 3a and 3b.

The storage section 18 comprises a body composition computing equation storage section which stores the following various body composition computing equations (1) to (7) for computing body composition indicators (i.e., subcutaneous fat thickness, abdominal muscle thickness, subcutaneous fat area, visceral fat area, total abdominal fat area, truncal fat percentage, and whole body fat percentage) based on both an impedance Zout-high based on the voltage Vout-high generated between the measuring electrodes 4a and 4b when the current Aout-high is passed between the first current electrodes 2a and 2b and an impedance Zin-low based on the voltage Vin-low generated between the measuring electrodes 4a and 4b when the current Ain-low is passed between the second current electrodes 3a and 3b. The storage section 18 also stores various other data.

```
subcutaneous fat thickness = 11.3 × Zin-low - 0.35 ×

Zout-high  ... (1)
```

```
abdominal muscle thickness = -0.031 × Zin-low + 0.101 ×

Zout-high  ... (2)
```

$$\text{subcutaneous fat area} = 23.5 \times \text{Zin-low} - 19.1 \times \text{Zout-high} \quad \dots \text{(3)}$$

$$\text{visceral fat area} = -4.1 \times \text{Zin-low} + 28.1 \times \text{Zout-high} \quad \dots \text{(4)}$$

$$\text{total abdominal fat area} = 28.8 \times \text{Zin-low} - 14.2 \times \text{Zout-high} \quad \dots \text{(5)}$$

$$\text{truncal fat percentage} = 1.61 + 1.32 \times \text{Zin-low} + 0.85 \times \text{Zout-high} \quad \dots \text{(6)}$$

$$\text{whole body fat percentage} = 0.85 \times (1.61 + 1.32 \times \text{Zin-low} + 0.85 \times \text{Zout-high}) + 1.1 \quad \dots \text{(7)}$$

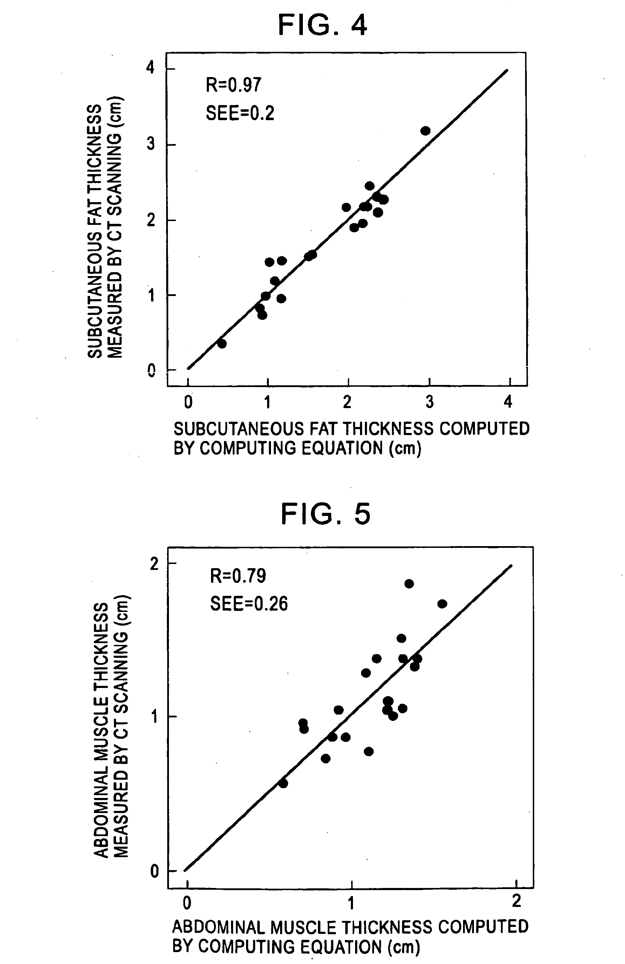

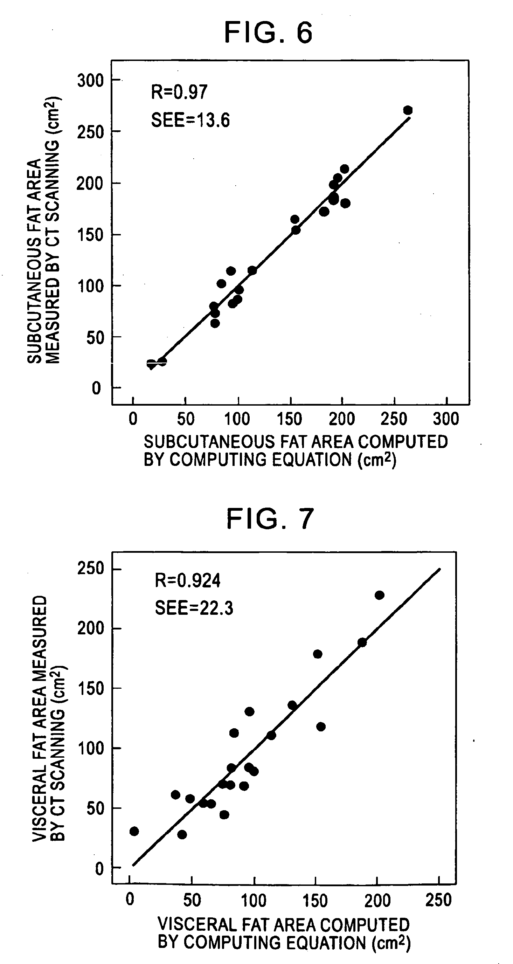

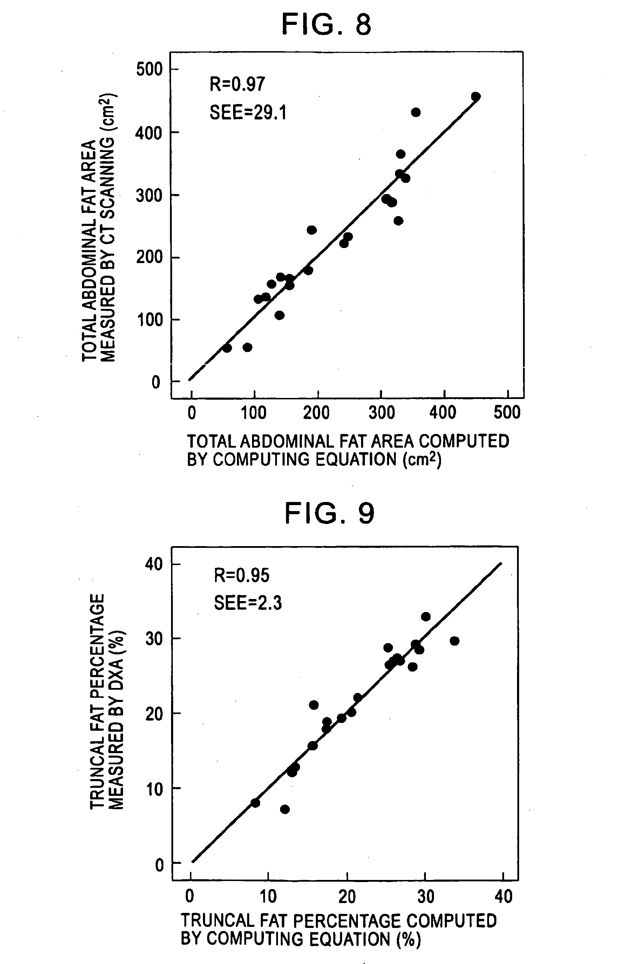

A subcutaneous fat thickness, an abdominal muscle thickness, a subcutaneous fat area, a visceral fat area and a total abdominal fat area which are computed by the above body composition computing equations (1) to (5) are highly correlated with a subcutaneous fat thickness, an abdominal muscle thickness, a subcutaneous fat area, a visceral fat area and a total abdominal fat area which are determined by CT (Computed Tomography) scanning which is generally considered to have good estimation accuracy, as shown in Figs. 4 to 8. Further, a truncal fat percentage and a whole body fat percentage which are computed by the above body composition computing equations (6) and (7) are highly correlated with a truncal fat percentage and a whole body fat percentage which are determined by DXA (Dual X-ray Absorptiometry) which is generally considered to have good estimation accuracy, as shown in Figs. 9 and 10.

The computing section 19 comprises an impedance computing section and a body composition computing section. The impedance computing section computes the impedances Zout-high and Zin-low based on the currents Aout-high and Ain-low generated from the current generating section 16 and the voltages Vout-high and Vin-low detected by the voltage detecting section 17, respectively. The body composition computing section computes body composition indicators (i.e., subcutaneous fat thickness, abdominal muscle thickness, subcutaneous fat area, visceral fat area, total abdominal fat area, truncal fat percentage, and whole body fat percentage) by substituting the impedances Zout-high and Zin-low computed by the impedance computing section into the body composition computing equations (1) to (7) stored in the body composition computing equation storage section. The computing section 19 also computes various other data.

The display section 12 displays the body composition indicators (i.e., subcutaneous fat thickness, abdominal muscle thickness, subcutaneous fat area, visceral fat area, total abdominal fat area, truncal fat percentage, and whole body fat percentage) computed by the body composition computing section, an estimated cross section of the abdomen of a body, and other input, measurement and result data.

The control section 20 (i) controls supply of electric power from the power section 14 to the sections constituting the electrical system of the present device, based on an ON signal from the power switch 13a, (ii) controls generation of the currents Aout-high and Ain-low from the current generating section 16, based on an ON signal from the measurement switch 13b, (iii) controls computations of the impedances Zout-high and Zin-low by the impedance computing section, based on the voltages Vout-high and Vin-low generated between the measuring electrodes 4a and 4b from the voltage detecting section 17, (iv) controls computations of the body composition indicators (i.e., subcutaneous fat thickness, abdominal muscle thickness, subcutaneous fat area, visceral fat area, total abdominal fat area, truncal fat percentage, and whole body fat percentage) by the body composition computing section, based on the impedances Zout-high and Zin-low computed by the impedance computing section and the body composition computing equations (1) to (7) stored in the body composition computing equation storage section, (v) controls activation of the driving motor 11 based on an ON signal from the measurement switch 13b, deactivation of the driving motor 11 based on an ON signal from the contact detecting sensors 10a and 10b, and computation of the width (body part width) between the left and right sides of the abdomen of a specific body part by the computing section 19 based on a detection signal from the encoders 9, (vi) controls display of various input, measurement and result data by the display section 12, in the input, measurement and result stages, and (vii) controls various other data.

The electrodes (the first current electrodes 2a and 2b, the second current electrodes 3a and 3b and the measuring electrodes 4a and 4b), the current generating section 16 and the voltage detecting section 17 constitute a body part impedance measuring section 21.

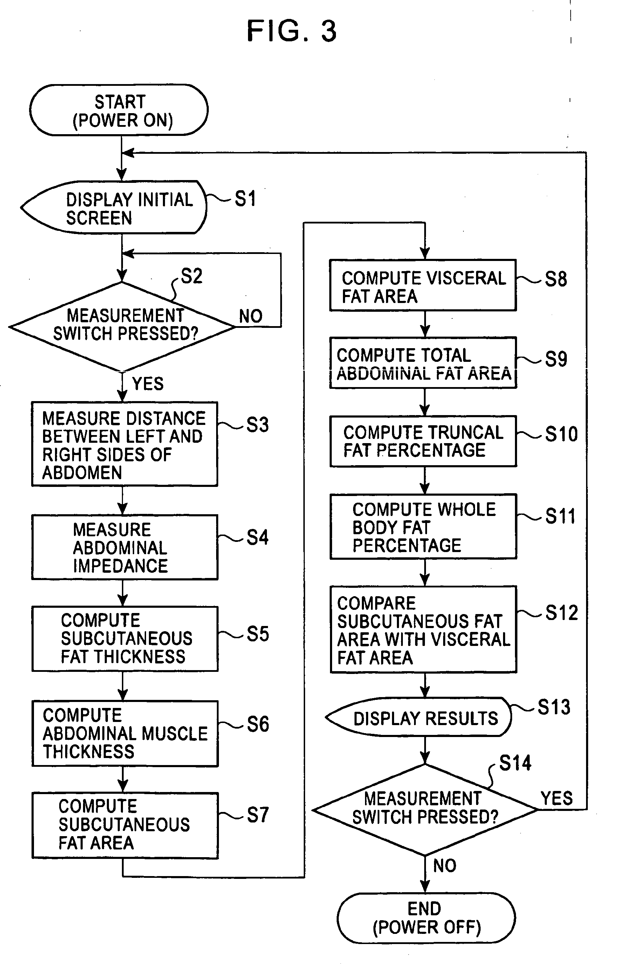

Next, the operations of the body composition meter (device which estimates body composition indicators in the abdomen) according to the present invention will be described by using primarily a flowchart shown in Fig. 3.

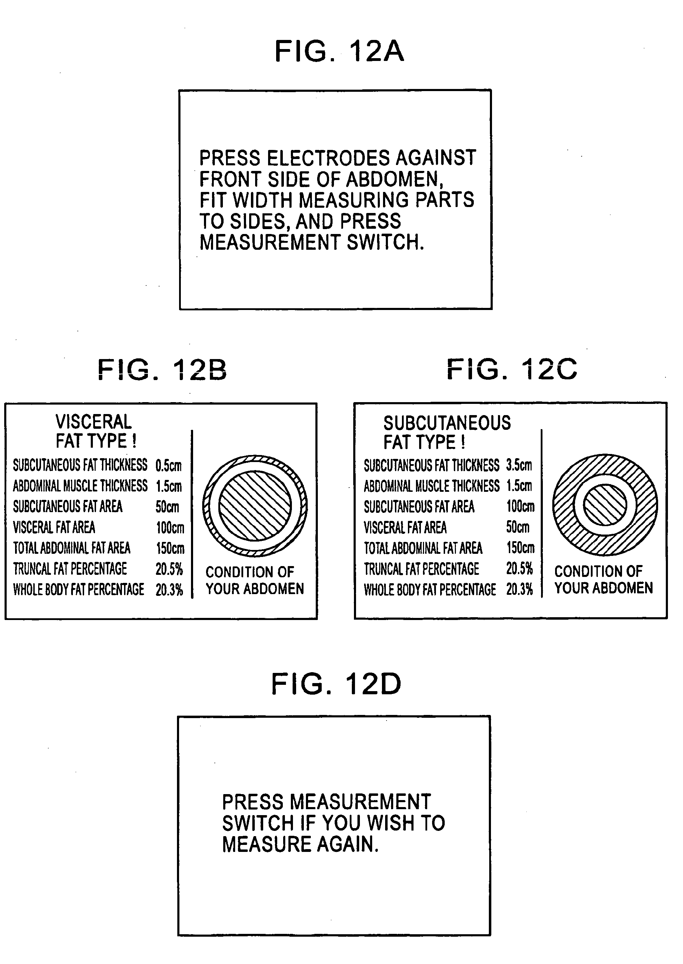

First, when the power switch 13a is pressed, electric power is supplied from the power section 14 to the sections in the electrical system, and an initial screen as shown in Fig. 12A is displayed on the display section 12 (STEP S1).

Then, it is determined in the control section 20 whether the measurement switch 13b has been pressed (STEP S2). If the measurement switch 13b has not been pressed (NO in STEP S2), this step of measurement standby state is repeated until the measurement switch 13b is pressed.

On the other hand, if the measurement switch 13b has been pressed with the adjustable arm 8 fitted around the abdomen 30 of a body such that the electrodes make close contact with the abdomen 30 (YES in STEP S2), the driving motor 11 is activated based on a control signal from the control section 20, and the two semi-horseshoe-shaped arms 8a and 8b thereby slide and contract automatically. Then, when the contact detecting sensors 10a and 10b detect that the arms 8a and 8b have made contact with the sides of the abdomen of the body, the driving motor 11 is deactivated based on a control signal from the control section 20, and the two semi-horseshoe-shaped arms 8a and 8b thereby stop sliding. Then, the stop position is detected by the electrodes which detect capacitance in the encoders 9. In other words, the distance when the adjustable arm 8 is fitted to the sides of the body is detected (STEP S3) .

Then, under control of the control section 20, the current generating section 16 selects the first current electrodes 2a and 2b and generates a high-frequency current Aout-high between the first current electrodes 2a and 2b, the voltage detecting section 17 detects a voltage Vout-high generated between the measuring electrodes 4a and 4b at that time, the impedance computing section computes an impedance Zout-high based on the current Aout-high generated from the current generating section 16 and the voltage Vout-high detected by the voltage detecting section 17, and the computed impedance Zout-high is stored in the storage section 18 temporarily. Then, under control of the control section 20, the current generating section 16 selects the second current electrodes 3a and 3b and generates a low-frequency current Ain-low between the second current electrodes 3a and 3b, the voltage detecting section 17 detects a voltage Vin-low generated between the measuring electrodes 4a and 4b at that time, the impedance computing section computes an impedance Zin-low based on the current Ain-low generated from the current generating section 16 and the voltage Vin-low detected by the voltage detecting section 17, and the impedance Zin-low is stored in the storage section 18 temporarily (STEP S4).

Then, under control of the control section 20, the body composition computing section computes a subcutaneous fat thickness by substituting the impedances Zout-high and Zin-low which are temporarily stored in the storage section 18 into the body composition computing equation (1) stored in the body composition computing equation storage section and stores the computed subcutaneous fat thickness in the storage section 18 temporarily (STEP S5).

Then, under control of the control section 20, the body composition computing section computes an abdominal muscle thickness by substituting the impedances Zout-high and Zin-low which are temporarily stored in the storage section 18 into the body composition computing equation (2) stored in the body composition computing equation storage section and stores the computed abdominal muscle thickness in the storage section 18 temporarily (STEP S6).

Then, under control of the control section 20, the body composition computing section computes a subcutaneous fat area by substituting the impedances Zout-high and Zin-low which are temporarily stored in the storage section 18 into the body composition computing equation (3) stored in the body composition computing equation storage section and stores the computed subcutaneous fat area in the storage section 18 temporarily (STEP S7).

Then, under control of the control section 20, the body composition computing section computes a visceral fat area by substituting the impedances Zout-high and Zin-low which are temporarily stored in the storage section 18 into the body composition computing equation (4) stored in the body composition computing equation storage section and stores the computed visceral fat area in the storage section 18 temporarily (STEP S8).

Then, under control of the control section 20, the body composition computing section computes a total abdominal fat area by substituting the impedances Zout-high and Zin-low which are temporarily stored in the storage section 18 into the body composition computing equation (5) stored in the body composition computing equation storage section and stores the computed total abdominal fat area in the storage section 18 temporarily (STEP S9).

Then, under control of the control section 20, the body composition computing section computes a truncal fat percentage by substituting the impedances Zout-high and Zin-low which are temporarily stored in the storage section 18 into the body composition computing equation (6) stored in the body composition computing equation storage section and stores the computed truncal fat percentage in the storage section 18 temporarily (STEP S10).

Then, under control of the control section 20, the body composition computing section computes a whole body fat percentage by substituting the impedances Zout-high and Zin-low which are temporarily stored in the storage section 18 into the body composition computing equation (7) stored in the body composition computing equation storage section and stores the computed whole body fat percentage in the storage section 18 temporarily (STEP S11).

Then, under control of the control section 20, the computing section 19 compares the subcutaneous fat area and visceral fat area stored temporarily in the storage section 18 with each other (STEP S12). If the difference between the visceral fat area and the subcutaneous fat area is larger than or equal to 0, the display section 12 displays measurement results with a message "VISCERAL FAT TYPE !" as shown in Fig. 12B. Meanwhile, if the difference between the visceral fat area and the subcutaneous fat area is not larger than or equal to 0, the display section 12 displays measurement results with a message "SUBCUTANEOUS FAT TYPE !" as shown in Fig. 12C for a given time period and then displays a screen for inquiring for remeasurement as shown in Fig. 12D (STEP S13).

Then, the control section 20 determines whether the measurement switch 13b has been pressed (STEP S14). If the measurement switch 13b has been pressed (YES in STEP S14), the display section 12 displays the initial screen (STEP S1) again, thereby making it possible to carry out the above series of measurement procedures. Meanwhile, if the measurement switch 13b has not been pressed even if the time keeping section has kept a certain length of time (NO in STEP S14), the power is turned off automatically, whereby the above series of operating procedures are ended.

According to the body composition meter of Example 1, the support 1 changes the distance between the current electrodes 2a and 2b, the distance between the current electrodes 3a and 3b, and the distance between the measuring electrodes 4a and 4b according to the width of the specific body part (i.e., the distance between the left and right sides of the abdomen). Thus, regardless of the width of the specific body part (i.e., the distance between the left and right sides of the abdomen), accurate impedances in the abdomen can be obtained, and body composition indicators can be estimated.

Example 2

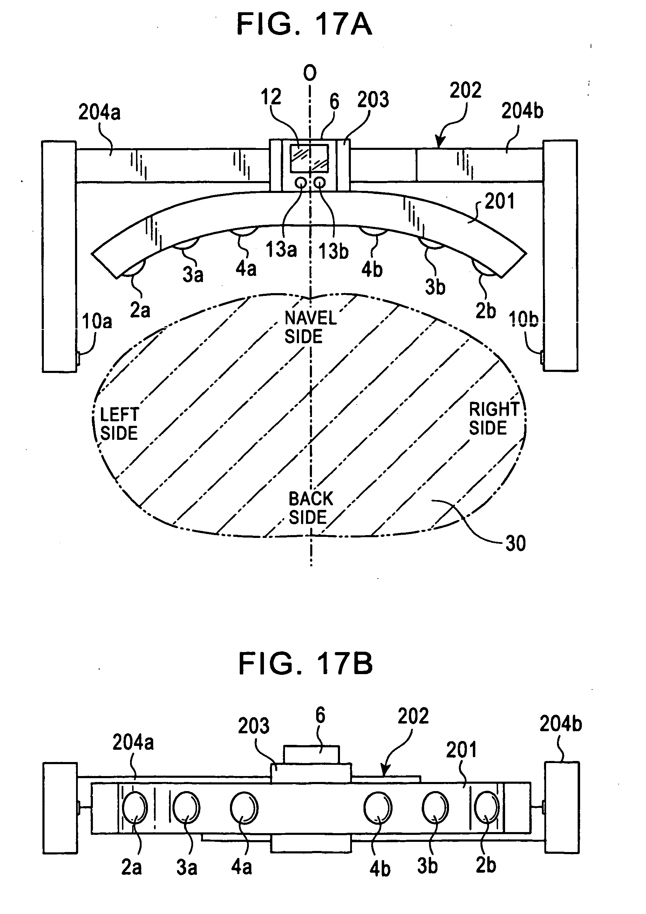

First, the specific constitution of a body composition meter (device which estimates body composition indicators in the abdomen by use of electrodes whose distance therebetween does not vary according to the width of the abdomen of a body) according to the present invention will be described by using primarily an external view shown in Fig. 17 and a block diagram shown in Fig. 2.

When viewed from outside, the body composition meter as Example 2 comprises a group of electrodes (first current electrodes 2a and 2b, second current electrodes 3a and 3b, and measuring electrodes 4a and 4b), an operation box 6, an adjustable member 202, and a support 201.

The support 201 supports the electrodes and has flexibility and a horseshoe shape so that the electrodes can make close contact with the abdomen 30 of a body.

The adjustable member 202 is a body part width acquiring section 7 which expands or contracts to measure the width of the abdomen of a body (i.e., the distance between the left and right sides of the abdomen). In other words, the adjustable member 202 comprises a detection driving box 203, adjustable arms 204 (two L-shaped arms 204a and 204b), and contact detecting sensors 10a and 10b, and the adjustable member 202 measures a position where the two L-shaped arms 204a and 204b make contact with the left and right sides of the abdomen of a body while passing through the detection driving box 203. More specifically, the contact detecting sensors 10a and 10b (such as photointerrupters and piezoelectric sensors) detect that the arms 204a and 204b are in contact with the left and right sides of the abdomen of a body and are provided on the inner sides of the ends of the two L-shaped arms 204a and 204b. The detection driving box 203 incorporates electrodes for detecting capacitance (portion of an encoder 9) and a driving motor 11 whose rotating shaft forms a pinion. Further, the two L-shaped arms 204a and 204b each have a rack-shaped portion which passes through the detection driving box 203, and the rack-shaped portion has electrodes for detecting capacitance (portion of the encoder 9). When the driving motor 11 drives to rotate the pinion-shaped rotating shaft with the rack-shaped portion engaging with the pinion-shaped portion in the detection driving box 203, the rack-shaped portion moves, and a position where the contact detecting sensors 10a and 10b have detected that the arms 204a and 204b have made contact with the left and right sides of the abdomen of the body is detected by the portion of the encoder 9 in the detection driving box 203 and the portion of the encoder 9 in the portions of the two L-shaped arms 204a and 204b which pass through the detection driving box 203.

The first current electrodes 2a and 2b are provided on both ends of the inner side (side which contacts the abdomen 30 of the body) of the support 201 to pass a current through the abdomen 30 of the body. The second current electrodes 3a and 3b are provided on the inner side (side which contacts the abdomen 30 of the body) of the support 201 and between the first current electrodes 2a and 2b to pass a current through the abdomen 30 of the body. The measuring electrodes 4a and 4b are provided on the inner side (side which contacts the abdomen 30 of the body) of the support 201 and between the second current electrodes 3a and 3b to detect a voltage generated by passing a current through the abdomen 30 of the body. The electrodes 2a, 3a and 4a and 2b, 3b and 4b are placed symmetrically with respect to the O axis such that the distance between the measuring electrodes 4a and 4b is 8 cm, the distance between the second current electrodes 3a and 3b is 16 cm to 20 cm (preferably 16 cm to 18 cm), and the distance between the first current electrodes 2a and 2b is 24 cm to 28 cm (preferably 24 cm). In other words, the electrodes 2a, 3a and 4a and 2b, 3b and 4b are placed symmetrically with respect to the O axis such that the distance between the measuring electrode 4a and the second current electrode 3a and between the measuring electrode 4b and the second current electrode 3b is 4 cm to 6 cm (preferably 4 cm to 5 cm), and the distance between the measuring electrode 4a and the first current electrode 2a and between the measuring electrode 4b and the first current electrode 2b is 8 cm to 10 cm (preferably 8 cm). The term "distance" used herein refers to the distance from the edge of one electrode to the edge of the other electrode.

The operation box 6 has a display section 12 and an input section 13 on the front face of a case and incorporates a power section 14, a time keeping section 15, a current generating section 16, a voltage detecting section 17, a storage section 18, a computing section 19 and a control section 20.

The power section 14 supplies electric power to the sections constituting the electrical system of the present device.

The time keeping section 15 keeps time.

The input section 13 comprises a power switch 13a and a measurement switch 13b. The power switch 13a is used to start supplying electric power from the power section 14. The measurement switch 13b is used to start measurement of impedance.

The current generating section 16 switches and selects between connections of the electrodes for passing a current (i.e., between connection of the first current electrodes 2a and 2b and connection of the second current electrodes 3a and 3b) and generates a current to be passed through the abdomen 30 of the body under control of the control section 20. The current generating section 16 generates a high-frequency current Aout-high (having a frequency of 128 kHz to 512 kHz, preferably a frequency of 256 kHz) for the first current electrodes 2a and 2b and generates a low-frequency current Ain-low (having a frequency of 4 kHz to 12.5 kHz, preferably a frequency of 5 kHz) for the second current electrodes 3a and 3b.

The voltage detecting section 17 detects a voltage Vout-high generated between the measuring electrodes 4a and 4b when the current Aout-high is passed between the first current electrodes 2a and 2b and a voltage Vin-low generated between the measuring electrodes 4a and 4b when the current Ain-low is passed between the second current electrodes 3a and 3b.

The storage section 18 comprises a body composition computing equation storage section which stores the following various body composition computing equations (8) to (14) for computing body composition indicators (i.e., subcutaneous fat thickness, abdominal muscle thickness, subcutaneous fat area, visceral fat area, total abdominal fat area, truncal fat percentage, and whole body fat percentage) based on both an impedance Zout-high based on the voltage Vout-high generated between the measuring electrodes 4a and 4b when the current Aout-high is passed between the first current electrodes 2a and 2b and an impedance Zin-low based on the voltage Vin-low generated between the measuring electrodes 4a and 4b when the current Ain-low is passed between the second current electrodes 3a and 3b. The storage section 18 also stores various other data.

subcutaneous fat thickness = a × Zin-low – b × Zout-high + c

x width of abdomen + d   ... (8)


abdominal muscle thickness = -e × Zin-low + f × Zout-high + g

+ width of abdomen + h   ... (9)


subcutaneous fat area = i × Zin-low – j × Zout-high + k × width

of abdomen + l   ... (10)

visceral fat area = -m × Zin-low + n × Zout-high + o × width of abdomen + p   ... (11)

total abdominal fat area = q × Zin-low - r × Zout-high + s × width of abdomen + t   ... (12)

truncal fat percentage = -u × (1/Zin-low) - v × (1/Zout-high) - w × width of abdomen + x   ... (13)

whole body fat percentage = 0.85 × {-u × (1/Zin-low) - v × (1/Zout-high) - w × width of abdomen + x} + 1.1   ... (14)

In the above formulae, a to x represent coefficients (constants).

The reason that the width of the abdomen (i.e., the distance between the left and right sides of the abdomen) is considered in the above body composition computing equations (8) to (14) unlike the embodiment (Example 1) in which the distances between the electrodes vary according to the width of the abdomen of a body is as follows. That is, since an abdominal portion with which the electrodes desirably make contact changes according to the size of the abdomen, an abdominal portion with which the electrodes actually make contact differs from the abdominal portion with which the electrodes desirably make contact in the embodiment (Example 2) in which the distances between the electrodes do not vary according to the width of the abdomen of a body, so that substantial errors may be included in measured data. Hence, the width of the abdomen is required to correct the errors. Meanwhile, in other words, in the embodiment (Example 1) in which the distances between the electrodes vary according to the width of the abdomen of a body, the width of the abdomen (i.e., the distance between the left and right sides of the abdomen) is not considered, because an abdominal portion with which the electrodes actually make contact is the same as an abdominal portion with which the electrodes desirably make contact and substantial errors therefore do not occur and correction of the errors is therefore not necessary.

The computing section 19 comprises an impedance computing section and a body composition computing section. The impedance computing section computes the impedances Zout-high and Zin-low based on the currents Aout-high and Ain-low generated from the current generating section 16 and the voltages Vout-high and Vin-low detected by the voltage detecting section 17, respectively. The body composition computing section computes body composition indicators (i.e., subcutaneous fat thickness, abdominal muscle thickness, subcutaneous fat area, visceral fat area, total abdominal fat area, truncal fat percentage, and whole body fat percentage) by substituting the impedances Zout-high and Zin-low computed by the impedance computing section into the body composition computing equations (8) to (14) stored in the body composition computing equation storage section. The computing section 19 also computes various other data.

The display section 12 displays the body composition indicators (i.e., subcutaneous fat thickness, abdominal muscle thickness, subcutaneous fat area, visceral fat area, total abdominal fat area, truncal fat percentage, and whole body fat percentage) computed by the body composition computing section, an estimated cross section of the abdomen of a body, and other input, measurement and result data.

The control section 20 (i) controls supply of electric power from the power section 14 to the sections constituting the electrical system of the present device, based on an ON signal from the power switch, (ii) controls generation of the currents Aout-high and Ain-low from the current generating section 16, based on an ON signal from the measurement switch 13b, (iii) controls computations of the impedances Zout-high and Zin-low by the impedance computing section, based on the voltages Vout-high and Vin-low generated between the measuring electrodes 4a and 4b from the voltage detecting section 17, (iv) controls computations of the body composition indicators (i.e., subcutaneous fat thickness, abdominal muscle thickness, subcutaneous fat area, visceral fat area, total abdominal fat area, truncal fat percentage, and whole body fat percentage) by the body composition computing section, based on the impedances Zout-high and Zin-low computed by the impedance computing section and the body composition computing equations (8) to (14) stored in the body composition computing equation storage section, (v) controls activation of the driving motor 11 based on an ON signal from the measurement switch 13b, deactivation of the driving motor 11 based on an ON signal from the contact detecting sensors 10a and 10b, and computation of the width (body part width) between the left and right sides of the abdomen of a specific body part by the computing section 19 based on a detection signal from the encoder 9, (vi) controls display of various input, measurement and result data by the display section 12, in the input, measurement and result stages, and (vii) controls various other data.

The electrodes (the first current electrodes 2a and 2b, the second current electrodes 3a and 3b and the measuring electrodes 4a and 4b), the current generating section 16 and the voltage detecting section 17 constitute a body part impedance measuring section.

Next, the operations of the body composition meter (device which estimates body composition indicators in the abdomen by use of electrodes whose distance therebetween does not vary according to the width of the abdomen of a body) according to the present invention will be described by using primarily a flowchart shown in Fig. 3.

First, when the power switch 13a is pressed, electric power is supplied from the power section 14 to the sections in the electrical system, and an initial screen as shown in Fig. 12A is displayed on the display section 12 (STEP S1).

Then, it is determined in the control section 20 whether the measurement switch 13b has been pressed (STEP S2). If the measurement switch 13b has not been pressed (NO in STEP S2), this step of measurement standby state is repeated until the measurement switch 13b is pressed.

On the other hand, if the measurement switch 13b has been pressed with the support 201 fitted around the abdomen 30 of a body such that the electrodes make close contact with the abdomen 30 (YES in STEP S2), the driving motor 11 is activated based on a control signal from the control section 20, and the two L-shaped arms 204a and 204b thereby slide and contract automatically. Then, when the contact detecting sensors 10a and 10b detect that the arms 204a and 204b have made contact with the sides of the abdomen of the body, the driving motor 11 is deactivated based on a control signal from the control section 20, and the two L-shaped arms 204a and 204b thereby stop sliding. Then, the stop position is detected by the electrodes which detect capacitance in the encoders 9. In other words, the distance when the adjustable member 202 is fitted to the sides of the body is detected (STEP S3).

Then, under control of the control section 20, the current generating section 16 selects the first current electrodes 2a and 2b and generates a high-frequency current Aout-high between the first current electrodes 2a and 2b, the voltage detecting section 17 detects a voltage Vout-high generated between the measuring electrodes 4a and 4b at that time, the impedance computing section computes an impedance Zout-high based on the current Aout-high generated from the current generating section 16 and the voltage Vout-high detected by the voltage detecting section 17, and the computed impedance Zout-high is stored in the storage section 18 temporarily. Then, under control of the control section 20, the current generating section 16 selects the second current electrodes 3a and 3b and generates a low-frequency current Ain-low between the second current electrodes 3a and 3b, the voltage detecting section 17 detects a voltage Vin-low generated between the measuring electrodes 4a and 4b at that time, the impedance computing section computes an impedance Zin-low based on the current Ain-low generated from the current generating section 16 and the voltage Vin-low detected by the voltage detecting section 17, and the computed impedance Zin-low is stored in the storage section 18 temporarily (STEP S4).

Then, under control of the control section 20, the body composition computing section computes a subcutaneous fat thickness by substituting the impedances Zout-high and Zin-low which are temporarily stored in the storage section 18 into the body composition computing equation (8) stored in the body composition computing equation storage section and stores the computed subcutaneous fat thickness in the storage section 18 temporarily (STEP S5).

Then, under control of the control section 20, the body composition computing section computes an abdominal muscle thickness by substituting the impedances Zout-high and Zin-low which are temporarily stored in the storage section 18 into the body composition computing equation (9) stored in the body composition computing equation storage section and stores the computed abdominal muscle thickness in the storage section 18 temporarily (STEP S6).

Then, under control of the control section 20, the body composition computing section computes a subcutaneous fat area by substituting the impedances Zout-high and Zin-low which are temporarily stored in the storage section 18 into the body composition computing equation (10) stored in the body composition computing equation storage section and stores the computed subcutaneous fat area in the storage section 18 temporarily (STEP S7).

Then, under control of the control section 20, the body composition computing section computes a visceral fat area by substituting the impedances Zout-high and Zin-low which are temporarily stored in the storage section 18 into the body composition computing equation (11) stored in the body composition computing equation storage section and stores the computed visceral fat area in the storage section 18 temporarily (STEP S8)

Then, under control of the control section 20, the body composition computing section computes a total abdominal fat area by substituting the impedances Zout-high and Zin-low which are temporarily stored in the storage section 18 into the body composition computing equation (12) stored in the body composition computing equation storage section and stores the computed total abdominal fat area in the storage section 18 temporarily (STEP S9).

Then, under control of the control section 20, the body composition computing section computes a truncal fat percentage by substituting the impedances Zout-high and Zin-low which are temporarily stored in the storage section 18 into the body composition computing equation (13) stored in the body composition computing equation storage section and stores the computed truncal fat percentage in the storage section 18 temporarily (STEP S10).

Then, under control of the control section 20, the body composition computing section computes a whole body fat percentage by substituting the impedances Zout-high and Zin-low which are temporarily stored in the storage section 18 into the body composition computing equation (14) stored in the body composition computing equation storage section and stores the computed whole body fat percentage in the storage section 18 temporarily (STEP S11).

Then, under control of the control section 20, the computing section 19 compares the subcutaneous fat area and visceral fat area stored temporarily in the storage section 18 with each other (STEP S12). If the difference between the visceral fat area and the subcutaneous fat area is larger than or equal to 0, the display section 12 displays measurement results with a message "VISCERAL FAT TYPE !" as shown in Fig. 12B. Meanwhile, if the difference between the visceral fat area and the subcutaneous fat area is not larger than or equal to 0, the display section 12 displays measurement results with a message "SUBCUTANEOUS FAT TYPE !" as shown in Fig. 12C for a given time period and then displays a screen for inquiring for remeasurement as shown in Fig. 12D (STEP S13).

Then, the control section 20 determines whether the measurement switch 13b has been pressed (STEP S14). If the measurement switch 13b has been pressed (YES in STEP S14), the display section 12 displays the initial screen (STEP S1) again, thereby making it possible to carry out the above series of measurement procedures. Meanwhile, if the measurement switch 13b has not been pressed even if the time keeping section has kept a certain length of time (NO in STEP S14), the power is turned off automatically, whereby the above series of operating procedures are ended.

According to the body composition meter of Example 2, body composition indicators are estimated in the body composition computing section merely by performing computations by substituting the width of a specific body part (i.e., the distance between the left and right sides of the abdomen) acquired (measured) by the body part width acquiring section (adjustable member 202) and impedances computed by the impedance computing section into the body composition computing equations stored in the body composition computing equation storage section. Thus, body composition indicators can be determined more accurately and more easily.

In the above Example 1, the body part width acquiring section 7 also serves as the support 1 and acquires the width of a body part (i.e., the distance between the left and right sides of the abdomen) by measurement. However, the distance between the left and right sides of the abdomen may be input through the input section 13.

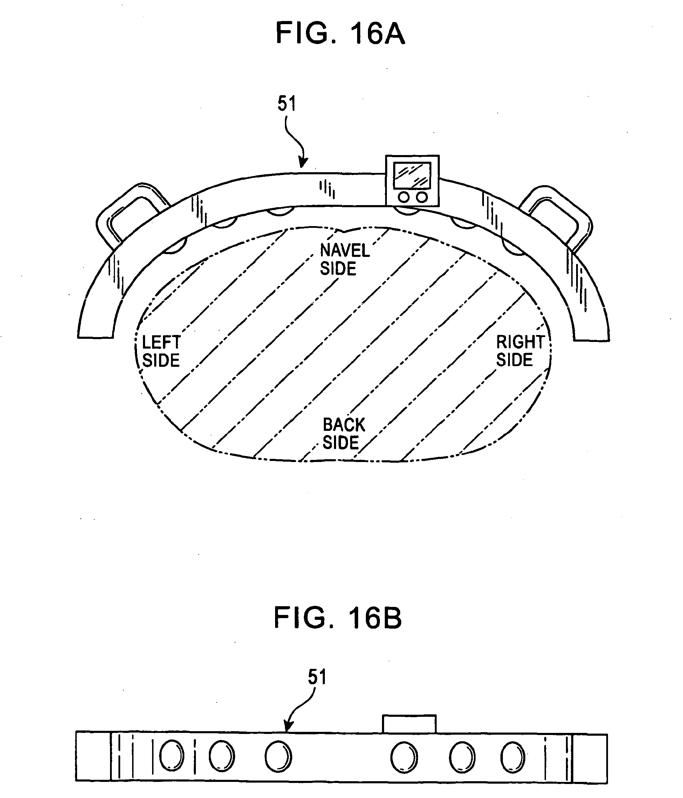

Further, in the above Example 1, the support 1 has a central portion which expands or contracts. However, the support 1 may have a central portion which does not expand or contract as shown in Fig. 16, at the sacrifice of accuracy.

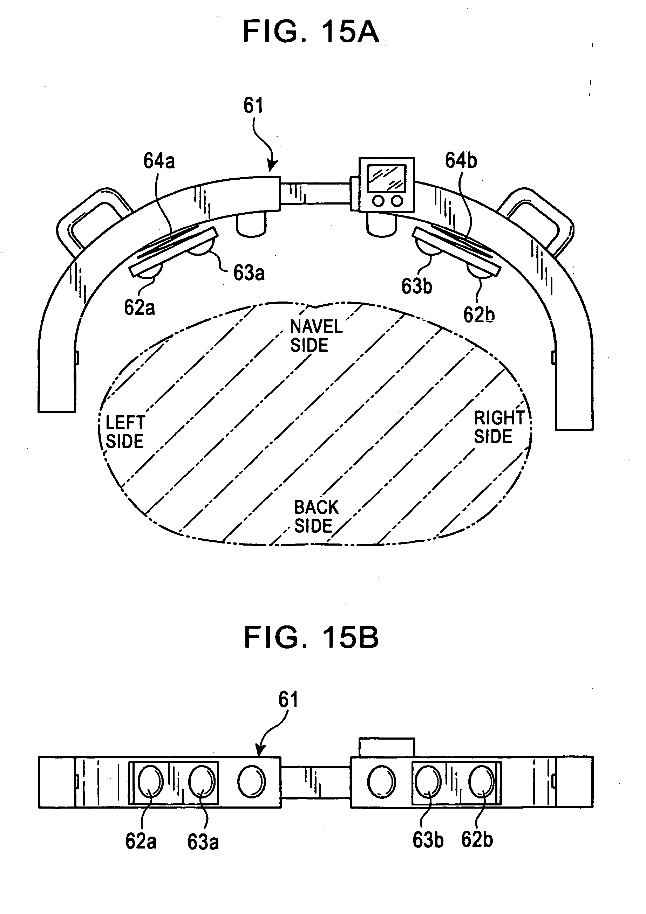

Further, in the above Example 1, the electrodes are disposed on the inner side of the support 1. However, flexible elastic members (such as springs and rubber) 64a and 64b may be provided between a support 61 and electrodes 62a, 62b, 63a and 63b as shown in Fig. 15 so that the electrodes provided on the support can make closer contact with the abdomen of a body when the support is applied to the abdomen of the body.

In the above Example 2, the width of the abdomen of a body (i.e., the distance between the left and right sides of the abdomen) is acquired through measurement using the adjustable member 202. However, the distance between the left and right sides of the abdomen may be input through the input section 13.

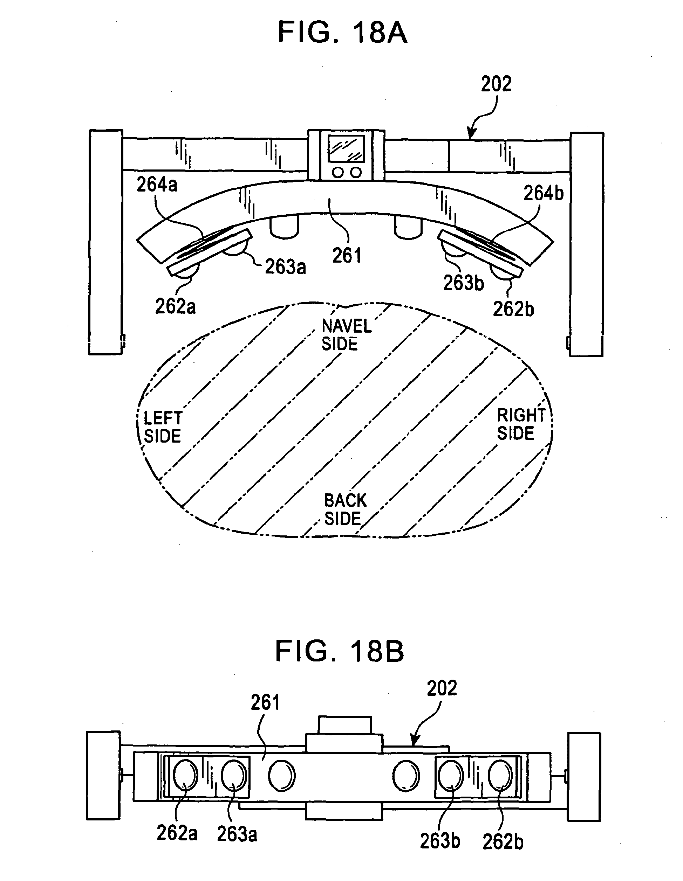

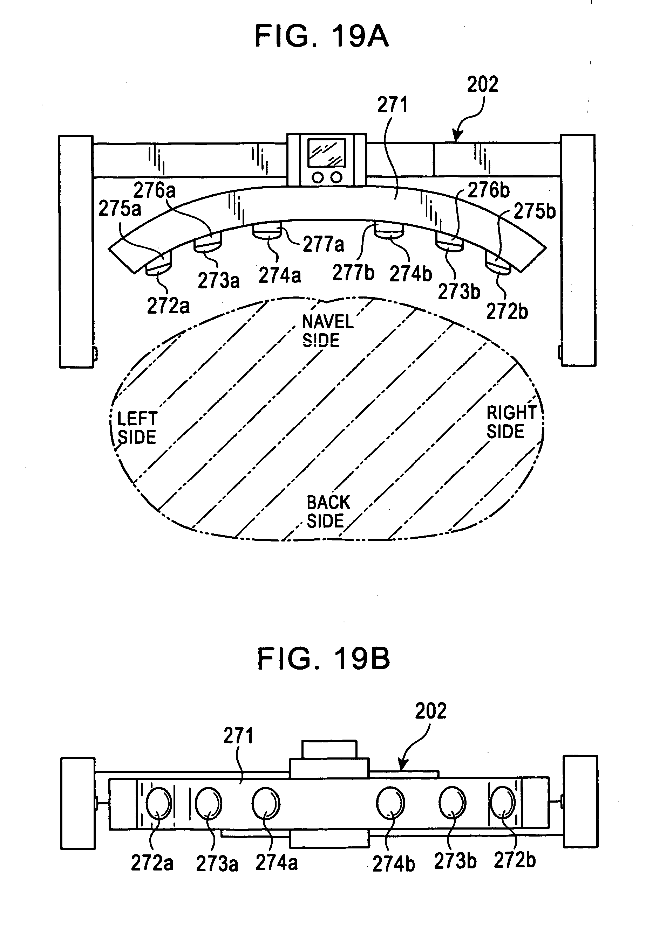

Further, in the above Example 2, the electrodes are disposed on the inner side of the support 201. However, flexible elastic members (such as springs and rubber) 264a and 264b may be provided between a support 261 and electrodes 262a, 262b, 263a and 263b as shown in Fig. 18 so that the electrodes provided on the support can make closer contact with the abdomen of a body when the support is applied to the abdomen of the body. Alternatively, flexible elastic members (such as springs and rubber) 275a, 275b, 275a, 275b, 275a and 275b may be provided between a support 271 and electrodes 272a, 272b, 273a, 273b, 274a and 274b as shown in Fig. 19

Further, in the above Examples 1 and 2, two pairs of current electrodes (first current electrodes 2a and 2b and second current electrodes 3a and 3b) are provided on the supports 1 and 201. However, three or more pairs of current electrodes may be provided on the supports. Further, the current electrodes may not have to be provided on the support and may be directly placed on a specific body part in the same arrangement as that on the support.

Further, in the above Examples 1 and 2, impedances in the abdomen 30 of the body are measured, and body composition indicators are estimated. However, it is also possible that impedances in arms, legs or the like of a body are measured and body composition indicators are estimated.

Further, in the above Examples 1 and 2, the electrodes are disposed in the circumferential direction of the abdomen of the body. However, the electrodes may be disposed in the longitudinal direction (body height direction) of the abdomen of the body.

Further, in the above Examples 1 and 2, the present body composition meter is applied to the abdomen of a human being. However, the present device may be modified to be applicable to a specific body part of an animal such as a dog or a cat.

**Claims**

1. A body composition meter comprising:

    multiple pairs of current electrodes (2a, 2b, 3a, 3b),
    a pair of measuring electrodes (4a, 4b),
    a current generating section (16),
    a voltage detecting section (17), and
    body composition estimating means (19),
    a body part width acquiring section (7),
    a display section (12), and
    a supports (1)
    wherein
    the multiple pairs of current electrodes are disposed on a specific body part sequentially at smaller spacings,
    the measuring electrodes are disposed between the innermost pair of current electrodes,
    the current generating section generates a current between each pair of current electrodes,
    the voltage detecting section detects a voltage generated between the measuring electrodes when the current generating section generates a current between each pair of current electrodes, and
    the body composition estimating means estimates body composition indicators based on all of the voltages detected by the voltage detecting section
    the body part width acquiring section acquires the width of the specific body part,
    the display section displays the specific body part width acquired by the body part width acquiring section as the width of the shape of a displayed cross section of the specific body part and displays the body composition indicators estimated by the body composition estimating means as the size of the inside of the displayed cross section of the specific body part at the magnification used to display the width of the specific body part, and
    the support supports the multiple pairs of current electrodes and the measuring electrodes to make the electrodes contact the specific body part.

2. The body composition meter according to claim 1, wherein the body composition estimating means comprises:

    an impedance computing section,
    a body composition computing equation storage section, and
    a body composition computing section,
    wherein
    the impedance computing section computes an impedance based on a current generated between each pair of current electrodes by the current generating section and a voltage detected by the voltage detecting section,
    the body composition computing equation storage section stores body composition computing equations for computing body composition indicators based on all of the impedances computed by the impedance computing section, and
    the body composition computing section computes body composition indicators by substituting the impedances computed by the impedance computing section into the body composition computing equations stored in the body composition computing equation storage section.

3. The body composition meter according to claim 1 or 2, wherein the current generating section generates a current such that the frequency of the current gradually lowers from the outermost pair of current electrodes to the innermost pair of current electrodes.

4. The body composition meter according to claim 3, wherein the current generating section generates a current of specific frequency ranging from 128 kHz to 512 kHz between the outermost pair of current electrodes and generates a current of specific frequency ranging from 4 kHz to 12.5 kHz between the innermost pair of current electrodes.

5. The body composition meter according to claim 4, wherein the specific frequency ranging from 128 kHz to 512 kHz is a frequency of 256 kHz, and the specific frequency ranging from 4 kHz to 12.5 kHz is a frequency of 5 kHz.

6. The body composition meter according to claim 1, wherein the support has a shape which fits the shape of the surface of the specific body part that makes contact with the electrodes and has a flexible portion at least in a portion thereof.

**7.** The body composition meter according to claim 1 or 6, wherein the support comprises:

> a body part width acquiring section, and
> a display section,
> wherein
> the body part width acquiring section measures the width of the specific body part, and
> the display section displays the specific body part width measured by the body part width acquiring section as the width of the shape of a displayed cross section of the specific body part and displays the body composition indicators estimated by the body composition estimating means as the size of the inside of the displayed cross section of the specific body part at the magnification used to display the width of the specific body part.

**8.** The body composition meter according to claim 7, wherein the body part width acquiring section comprises:

> an adjustable arm, and
> encoders,
> wherein
> the adjustable arm expands or contracts in the width direction of the specific body part to fit around the specific body part, and
> the encoders detect the distance when the adjustable arm is fitted around the specific body part.

**9.** The body composition meter according to claim 8, wherein the body part width acquiring section further comprises:

> contact detecting sensors, and
> a driving motor,
> wherein
> the contact detecting sensors detect that the adjustable arm is fitted around the specific body part, and
> the driving motor expands or contracts the adjustable arm and stops the adjustable arm at positions where the contact detecting sensors detect that the adjustable arm is fitted around the specific body part.

**10.** The body composition meter according to any one of claims 1 to 9, wherein the specific body part is the abdomen of a body, and the body composition indicator is at least one selected from the group consisting of a subcutaneous fat thickness, an abdominal muscle thickness, a subcutaneous fat area, a visceral fat area, a total abdominal fat area, a truncal fat percentage and a whole body fat percentage.

**11.** The body composition meter according to claim 10, wherein the distance between the multiple pairs of current electrodes and the measuring electrodes is a specific distance ranging from 4 cm to 10 cm.

**12.** The body composition meter according to claim 11, wherein the specific distance between the outermost pair of current electrodes and the measuring electrodes is 8 cm, and the specific distance between the innermost pair of current electrodes and the measuring electrodes is 4 cm to 5 cm.

**13.** The body composition meter according to claim 11 or 12, wherein the distance between the measuring electrodes is 8 cm.

**14.** The body composition meter according to claim 1, wherein the support changes the distances between ones of the current electrodes and the others of the current electrodes and the distance between one of the measuring electrodes and the other of the measuring electrodes according to the width of the specific body part.

**15.** The body composition meter according to claim 1, further comprising a body part width acquiring section which acquires the width of the specific body part,

> the body composition estimating means comprising:

>> an impedance computing section,
>> a body composition computing equation storage section, and
>> a body composition computing section,
>> wherein
>> the impedance computing section computes an impedance based on a current generated between each pair of current electrodes by the current generating section and a voltage detected by the voltage detecting section,

the body composition computing equation storage section stores body composition computing equations for computing body composition indicators based on the width of the specific body part acquired by the body part width acquiring section and all of the impedances computed by the impedance computing section, and

the body composition computing section computes body composition indicators by substituting the impedances computed by the impedance computing section into the body composition computing equations stored in the body composition computing equation storage section.

16. The body composition meter according to claim 15, wherein the current generating section generates a current such that the frequency of the current gradually lowers from the outermost pair of current electrodes to the innermost pair of current electrodes.

17. The body composition meter according to claim 16, wherein the current generating section generates a current of specific frequency ranging from 128 kHz to 512 kHz between the outermost pair of current electrodes and generates a current of specific frequency ranging from 4 kHz to 12.5 kHz between the innermost pair of current electrodes.

18. The body composition meter according to claim 17, wherein the specific frequency ranging from 128 kHz to 512 kHz is a frequency of 256 kHz, and the specific frequency ranging from 4 kHz to 12.5 kHz is a frequency of 5 kHz.


**Patentansprüche**

1. Körperzusammensetzungs-Messvorrichtung, die umfasst:

mehrere Paare von Stromelektroden (2a, 2b, 3a, 3b),
ein Paar Messelektroden (4a, 4b)
einen Stromerzeugungsabschnitt (16),
einen Spannungsermittlungsabschnitt (17), und
eine Einrichtung (19) zum Schätzen der Körperzusammensetzung,
einen Abschnitt (7) zum Erfassen der Breite eines Körperteils,
einen Anzeigeabschnitt (12); und
einen Träger (1),
wobei
die mehreren Paare von Stromelektroden an einem bestimmten Körperteil sequenziell in kleineren Abständen angeordnet sind,
die Messelektroden zwischen dem innersten Paar von Stromelektroden angeordnet sind,
der Stromerzeugungsabschnitt einen Strom zwischen jedem Paar Stromelektroden erzeugt,
der Spannungserfassungsabschnitt eine Spannung erfasst, die zwischen den Messelektroden erzeugt wird, wenn der Stromerzeugungsabschnitt einen Strom zwischen jedem Paar Stromelektroden erzeugt, und
die Einrichtung zum Schätzen der Körperzusammensetzungs-Indikatoren auf Basis aller durch den Spannungserfassungsabschnitt erfassten Spannungen schätzt,
der Abschnitt zum Erfassen der Breite eines Körperteils die Breite des bestimmten Körperteils erfasst,
der Anzeigeabschnitt die Breite des bestimmten Körperteils, die durch den Abschnitt zum Erfassen der Breite eines Körperteils erfasst wird, als die Breite der Form eines angezeigten Querschnitts des bestimmten Körperteils anzeigt und die die Körperzusammensetzungs-Indikatoren, die durch die Einrichtung zum Schätzen der Körperzusammensetzung geschätzt werden, als die Größe des Inneren des angezeigten Querschnitts des bestimmten Körperteils bei der Vergrößerung anzeigt, die verwendet wird, um die Breite des bestimmten Körperteils anzuzeigen, und
der Träger die mehreren Paare von Stromelektroden und die Messelektroden so trägt, dass die Elektroden in Kontakt mit dem bestimmten Körperteil sind.

2. Körperzusammensetzungs-Messvorrichtung nach Anspruch 1, wobei die Einrichtung zum Schätzen der Körperzusammensetzung umfasst:

einen Impedanz-Berechnungsabschnitt,
einen Abschnitt zum Speichern von Körperzusammensetzungs-Berechnungsgleichungen, und

einen Körperzusammensetzungs-Berechnungsabschnitt,
wobei
der Impedanz-Berechnungsabschnitt eine Impedanz auf Basis eines Stroms, der zwischen jedem Paar Stromelektroden durch den Stromerzeugungsabschnitt erzeugt wird, und einer Spannung berechnet, die durch den Spannungserfassungsabschnitt erfasst wird,
der Abschnitt zum Speichern von Körperzusammensetzungs-Berechnungsgleichungen Körperzusammensetzungs-Berechnungsgleichungen zum Berechnen von Körperzusammensetzungs-Indikatoren auf Basis aller durch den Impedanz-Berechnungsabschnitt berechneten Impedanzen speichert, und
der Körperzusammensetzungs-Berechnungsabschnitt Körperzusammensetzungs-Indikatoren berechnet, indem er die durch den Impedanz-Berechnungsabschnitt berechneten Impedanzen in die in dem Abschnitt zum Speichern von Körperzusammensetzungs-Berechnungsgleichungen gespeicherten Körperzusammensetzungs-Berechnungsgleichungen einsetzt.

3. Körperzusammensetzungs-Messvorrichtung nach Anspruch 1 oder 2, wobei der Stromerzeugungsabschnitt einen Strom so erzeugt, dass die Frequenz des Stroms von dem äußersten Paar von Stromelektroden zum innersten Paar von Stromelektroden allmählich abnimmt.

4. Körperzusammensetzungs-Messvorrichtung nach Anspruch 3, wobei der Stromerzeugungsabschnitt einen Strom einer bestimmten Frequenz, die von 128 kHz bis 512 kHz zwischen dem äußersten Paar von Stromelektroden erzeugt, und einen Strom einer bestimmten Frequenz, die von 4 kHz bis 12,5 kHz reicht, zwischen dem innersten Paar von Stromelektroden erzeugt.

5. Körperzusammensetzungs-Messvorrichtung nach Anspruch 4, wobei die bestimmte Frequenz, die von 128 kHz bis 512 kHz reicht, eine Frequenz von 256 kHz ist und die bestimmte Frequenz, die von 4 kHz bis 12,5 kHz reicht, eine Frequenz von 5 kHz ist.

6. Körperzusammensetzungs-Messeinrichtung nach Anspruch 1, wobei der Träger eine Form hat, die zu der Form der Oberfläche des bestimmten Körperteils passt, der mit den Elektroden in Kontakt ist, und einen flexiblen Abschnitt in wenigstens einem Abschnitt desselben aufweist.

7. Körperzusammensetzungs-Messvorrichtung nach Anspruch 1 oder 6, wobei der Träger umfasst:

einen Abschnitt zum Erfassen der Breite eines Körperteils, und
einen Anzeigeabschnitt,
wobei
der Abschnitt zum Erfassen der Breite eines Körperteils die Breite des bestimmten Körperteils misst, und
der Anzeigeabschnitt die Breite des bestimmten Körperteils, die durch den Abschnitt zum Erfassen der Breite eines Körperteils gemessen wird, als die Breite der Form eines angezeigten Querschnitts des bestimmten Körperteils anzeigt und die Körperzusammensetzungs-Indikatoren, die durch die Körperzusammensetzungs-Schätzeinrichtung geschätzt werden, als die Größe des Inneren des angezeigten Querschnitts des bestimmten Körperteils bei der Vergrößerung anzeigt, die verwendet wird, um die Breite des bestimmten Körperteils anzuzeigen.

8. Körperzusammensetzungs-Messvorrichtung nach Anspruch 7, wobei der Abschnitt zum Erfassen der Breite eines Körperteils umfasst:

einen verstellbaren Arm, und
Codiereinrichtungen,
wobei
der verstellbare Arm in der Breitenrichtung des bestimmten Körperteils ausfährt oder einfährt, um um den bestimmten Körperteil herum zu passen, und
die Codiereinrichtungen den Abstand erfassen, wenn der verstellbare Arm um den bestimmten Körperteil herum gepasst ist.

9. Körperzusammensetzungs-Messvorrichtung nach Anspruch 8, wobei der Abschnitt zum Erfassen einer Körperteil-Breite des Weiteren umfasst:

Kontakt-Erfassungssensoren, und

einen Antriebsmotor,

wobei

die Kontakt-Erfassungssensoren erfassen, dass der verstellbare Arm um den bestimmten Körperteil herum gepasst ist, und

der Antriebsmotor den verstellbaren Arm ausfährt oder einzieht und den verstellbaren Arm an Positionen anhält, an denen die Kontakt-Erfassungssensoren erfassen, dass der verstellbare Arm um den bestimmten Körperteil herum gepasst ist.

10. Körperzusammensetzungs-Messvorrichtung nach einem der Ansprüche 1 bis 9, wobei der bestimmte Körperteil der Bauch eines Körpers ist und der Körperzusammensetzungs-Indikator wenigstens einer ist, der aus der Gruppe ausgewählt wird, die aus einer Dicke von subkutanem Fett, einer Dicke des Bauchmuskels, einer Fläche von subkutanem Fett, einer Fläche von Eingeweidefett, einer Gesamtfläche von Bauchfett, einem Rumpffett-Anteil und einem Gesamt-Körperfett-Anteil besteht.

11. Körperzusammensetzungs-Messeinrichtung nach Anspruch 10, wobei der Abstand zwischen den mehreren Paaren von Stromelektroden und den Messelektroden ein bestimmter Abstand ist, der von 4 cm bis 10 cm reicht.

12. Körperzusammensetzungs-Messeinrichtung nach Anspruch 11, wobei der bestimmte Abstand zwischen dem äußersten Paar von Stromelektroden und den Messelektroden 8 cm beträgt und der bestimmte Abstand zwischen dem innersten Paar von Stromelektroden und den Messelektroden 4 cm bis 5 cm beträgt.

13. Körperzusammensetzungs-Messeinrichtung nach Anspruch 11 oder 12, wobei der Abstand zwischen den Messelektroden 8 cm beträgt.

14. Körperzusammensetzungs-Messeinrichtung nach Anspruch 1, wobei der Träger die Abstände zwischen den einen Stromelektroden und den anderen Stromelektroden sowie den Abstand zwischen einer der Messelektroden und der anderen der Messelektroden entsprechend der Breite des bestimmten Körperteils ändert.

15. Körperzusammensetzungs-Messvorrichtung nach Anspruch 1, die des Weiteren einen Abschnitt zum Erfassen der Breite eines Körperteils umfasst, der die Breite des bestimmten Körperteils erfasst, wobei die Körperzusammensetzungs-Schätzeinrichtung umfasst:

einen Impedanz-Berechnungsabschnitt,

einen Abschnitt zum Speichern von Körperzusammensetzungs-Berechnungsgleichungen, und

einen Körperzusammensetzungs-Berechnungsabschnitt,

wobei

der Impedanz-Berechnungsabschnitt eine Impedanz auf Basis eines Stroms, der zwischen jedem Paar Stromelektroden durch den Stromerzeugungsabschnitt erzeugt wird, und einer Spannung berechnet, die durch den Spannungsermittlungsabschnitt ermittelt wird

der Abschnitt zum Speichern von Körperzusammensetzungs-Berechnungsgleichungen Körperzusammensetzungs-Berechnungsgleichungen zum Berechnen von Körperzusammensetzungs-Indikatoren auf Basis der durch den Abschnitt zum Erfassen der Breite eines Körperteils erfassten Breite des bestimmten Körperteils und aller durch den Impedanz-Berechnungsabschnitt berechneten Impedanzen speichert, und

der Körperzusammensetzungs-Berechnungsabschnitt Körperzusammensetzungs-Indikatoren berechnet, indem er die durch den Impedanz-Berechnungsabschnitt berechneten Impedanzen in die in dem Abschnitt zum Speichern von Körperzusammensetzungs-Berechnungsgleichungen gespeicherten Körperzusammensetzungs-Berechnungsgleichungen einsetzt.

16. Körperzusammensetzungs-Messvorrichtung nach Anspruch 15, wobei der Stromerzeugungsabschnitt einen Strom so erzeugt, dass die Frequenz des Stroms von dem äußersten Paar von Stromelektroden zu dem innersten Paar von Stromelektroden allmählich abnimmt.

17. Körperzusammensetzungs-Messvorrichtung nach Anspruch 16, wobei der Stromerzeugungsabschnitt einen Strom einer bestimmten Frequenz, die von 128 kHz bis 512 kHz reicht, zwischen dem äußersten Paar von Stromelektroden erzeugt und einen Strom einer bestimmten Frequenz, die von 4 kHz bis 12,5 kHz reicht, zwischen dem innersten Paar von Stromelektroden erzeugt.

18. Körperzusammensetzungs-Messvorrichtung nach Anspruch 17, wobei die bestimmte Frequenz, die von 128 kHz bis 512 kHz reicht, eine Frequenz von 256 kHz ist und die bestimmte Frequenz, die von 4 kHz bis 12,5 kHz reicht, eine Frequenz von 5 kHz ist.

## Revendications

1. Dispositif de mesure de composition corporelle comprenant :

de multiples paires d'électrodes de courant (2a, 2b, 3a, 3b),
une paire d'électrodes de mesure (4a, 4b),
une section de génération de courant (16),
une section de détection de tension (17), et
un moyen d'estimation de composition corporelle (19),
une section d'acquisition de largeur de partie corporelle (7),
une section d'affichage (12), et
un support (1)
où
les multiples paires d'électrodes de courant sont disposées sur une partie corporelle spécifique séquentiellement au niveau d'espaces plus petits,
les électrodes de mesure sont disposées entre la paire la plus à l'intérieur d'électrodes de courant,
la section de génération de courant génère un courant entre chaque paire d'électrodes de courant,
la section de détection de tension détecte une tension générée entre les électrodes de mesure lorsque la section de génération de courant génère un courant entre chaque paire d'électrodes de courant, et
le moyen d'estimation de composition corporelle estime des indicateurs de composition corporelle sur la base de toutes les tensions détectées par la section de détection de tension,
la section d'acquisition de largeur de partie corporelle acquiert la largeur de la partie corporelle spécifique,
la section d'affichage affiche la largeur de partie corporelle spécifique acquise par la section d'acquisition de largeur de partie corporelle en tant que largeur de la forme d'une section transversale affichée de la partie corporelle spécifique et affiche les indicateurs de composition corporelle estimés par le moyen d'estimation de composition corporelle en tant que taille de l'intérieur de la section transversale affichée de la partie corporelle spécifique au grossissement utilisé pour afficher la largeur de la partie corporelle spécifique, et
le support supporte les multiples paires d'électrodes de courant et les électrodes de mesure pour amener les électrodes à venir en contact avec la partie corporelle spécifique.

2. Dispositif de mesure de composition corporelle selon la revendication 1, dans lequel le moyen d'estimation de composition corporelle comprend :

une section de calcul d'impédance,
une section de mémorisation d'équations de calcul de composition corporelle, et
une section de calcul de composition corporelle,
où
la section de calcul d'impédance calcule une impédance sur la base d'un courant généré entre chaque paire d'électrodes de courant par la section de génération de courant et d'une tension détectée par la section de détection de tension,
la section de mémorisation d'équations de calcul de composition corporelle mémorise les équations de calcul de composition corporelle afin de calculer les indicateurs de composition corporelle sur la base de toutes les impédances calculées par la section de calcul d'impédance, et
la section de calcul de composition corporelle calcule les indicateurs de composition corporelle en substituant les impédances calculées par la section de calcul d'impédance dans les équations de calcul de composition corporelle mémorisées dans la section de mémorisation d'équations de calcul de composition corporelle.

3. Dispositif de mesure de composition corporelle selon la revendication 1 ou 2, dans lequel la section de génération de courant génère un courant de sorte que la fréquence du courant diminue progressivement depuis la paire la plus à l'extérieur des électrodes de courant jusqu'à la paire la plus à l'intérieur des électrodes de courant.

4. Dispositif de mesure de composition corporelle selon la revendication 3, dans lequel la section de génération de courant génère un courant de fréquence spécifique allant de 128 kHz à 512 kHz entre la paire la plus à l'extérieur des électrodes de courant et génère un courant de fréquence spécifique allant de 4 kHz à 12,5 kHz entre la paire la plus à l'intérieur des électrodes de courant.

**5.** Dispositif de mesure de composition corporelle selon la revendication 4, dans lequel la fréquence spécifique allant de 128 kHz à 512 kHz est une fréquence de 256 kHz et la fréquence spécifique allant de 4 kHz à 12,5 kHz est une fréquence de 5 kHz.

**6.** Dispositif de mesure de composition corporelle selon la revendication 1, dans lequel le support présente une forme qui s'adapte à la forme de la surface de la partie corporelle spécifique qui vient en contact avec les électrodes et comporte une partie flexible au niveau d'au moins une partie de celui-ci.

**7.** Dispositif de mesure de composition corporelle selon la revendication 1 ou 6, dans lequel le support comprend :

une section d'acquisition de largeur de partie corporelle, et
une section d'affichage,
où
la section d'acquisition de largeur de partie corporelle mesure la largeur de la partie corporelle spécifique, et
la section d'affichage affiche la largeur de partie corporelle spécifique mesurée par la section d'acquisition de largeur de partie corporelle en tant que largeur de la forme d'une section transversale affichée de la partie corporelle spécifique et affiche les indicateurs de composition corporelle estimés par le moyen d'estimation de composition corporelle en tant que taille de l'intérieur de la section transversale affichée de la partie corporelle spécifique au grossissement utilisé pour afficher la largeur de la partie corporelle spécifique.

**8.** Dispositif de mesure de composition corporelle selon la revendication 7, dans lequel la section d'acquisition de largeur de partie corporelle comprend :

un bras réglable, et
des codeurs,
où
le bras réglable s'étend ou se contracte dans la direction de la largeur de la partie corporelle spécifique pour s'adapter autour de la partie corporelle spécifique, et
les codeurs détectent la distance lorsque le bras réglable est adapté autour de la partie corporelle spécifique.

**9.** Dispositif de mesure de composition corporelle selon la revendication 8, dans lequel la section d'acquisition de largeur de partie corporelle comprend en outre :

des capteurs de détection de contact, et
un moteur d'entraînement,
où
les capteurs de détection de contact détectent que le bras réglable est adapté autour de la partie corporelle spécifique, et
le moteur d'entraînement étend ou contracte le bras réglable et arrête le bras réglable à des positions où les capteurs de détection de contact détectent que le bras réglable est adapté autour de la partie corporelle spécifique.

**10.** Dispositif de mesure de composition corporelle selon l'une quelconque des revendications 1 à 9, dans lequel la partie corporelle spécifique est l'abdomen d'un corps et l'indicateur de composition corporelle est au moins un élément sélectionné à partir du groupe constitué d'une épaisseur de graisse sous-cutanée, d'une épaisseur de muscle abdominal, d'une surface de graisse sous-cutanée, d'une surface de graisse viscérale, d'une surface de graisse abdominale totale, d'un pourcentage de graisse du tronc et d'un pourcentage de graisse du corps tout entier.

**11.** Dispositif de mesure de composition corporelle selon la revendication 10, dans lequel la distance entre les multiples paires d'électrodes de courant et les électrodes de mesure est une distance spécifique allant de 4 cm à 10 cm.

**12.** Dispositif de mesure de composition corporelle selon la revendication 11, dans lequel la distance spécifique entre la paire la plus à l'extérieur des électrodes de courant et les électrodes de mesure est de 8 cm et la distance spécifique entre la paire la plus à l'intérieur des électrodes de courant et des électrodes de mesure est de 4 cm à 5 cm.

**13.** Dispositif de mesure de composition corporelle selon la revendication 11 ou 12, dans lequel la distance entre les électrodes de mesure est de 8 cm.

**14.** Dispositif de mesure de composition corporelle selon la revendication 1, dans lequel le support modifie les distances entre certaines des électrodes de courant et les autres électrodes des électrodes de courant et la distance entre l'une des électrodes de mesure et l'autre des électrodes de mesure conformément à la largeur de la partie corporelle spécifique.

**15.** Dispositif de mesure de composition corporelle selon la revendication 1, comprenant en outre une section d'acquisition de largeur de partie corporelle qui acquiert la largeur de la partie corporelle spécifique,

le moyen d'estimation de composition corporelle comprenant :

une section de calcul d'impédance,
une section de mémorisation d'équations de calcul de composition corporelle, et
une section de calcul de composition corporelle,
où
la section de calcul d'impédance calcule une impédance sur la base d'un courant généré entre chaque paire d'électrodes de courant par la section de génération de courant et d'une tension détectée par la section de détection de tension,
la section de mémorisation d'équations de calcul de composition corporelle mémorise des équations de calcul de composition corporelle afin de calculer des indicateurs de composition corporelle sur la base de la largeur de la partie corporelle spécifique acquise par la section d'acquisition de largeur de partie corporelle et de toutes les impédances calculées par la section de calcul d'impédance, et
la section de calcul de composition corporelle calcule les indicateurs de composition corporelle en substituant les impédances calculées par la section de calcul d'impédance dans les équations de calcul de composition corporelle mémorisées dans la section de mémorisation d'équations de calcul de composition corporelle.

**16.** Dispositif de mesure de composition corporelle selon la revendication 15, dans lequel la section de génération de courant génère un courant de sorte que la fréquence du courant diminue progressivement depuis la paire la plus à l'extérieur des électrodes de courant jusqu'à la paire la plus à l'intérieur des électrodes de courant.

**17.** Dispositif de mesure de composition corporelle selon la revendication 16, dans lequel la section de génération de courant génère un courant de fréquence spécifique allant de 128 kHz à 512 kHz entre la paire la plus à l'extérieur des électrodes de courant et génère un courant de fréquence spécifique allant dé 4 kHz à 12,5 kHz entre la paire la plus à l'intérieur des électrodes de courant.

**18.** Dispositif de mesure de composition corporelle selon la revendication 17, dans lequel la fréquence spécifique allant de 128 kHz à 512 kHz est une fréquence de 256 kHz et la fréquence spécifique allant de 4 kHz à 12,5 kHz est une fréquence de 5 kHz.

# FIG. 1A

# FIG. 1B

# FIG. 2

# FIG. 3

```
          START
        (POWER ON)
             │
             ▼
    DISPLAY INITIAL  ──S1
       SCREEN
             │
             ▼
      ┌──S2
   MEASUREMENT        NO
 SWITCH PRESSED? ────────
             │
            YES
             │
             ▼
  MEASURE DISTANCE   ──S3
  BETWEEN LEFT AND
  RIGHT SIDES OF
  ABDOMEN
             │
             ▼
     MEASURE          ──S4
   ABDOMINAL
   IMPEDANCE
             │
             ▼
    COMPUTE           ──S5
  SUBCUTANEOUS
  FAT THICKNESS
             │
             ▼
   COMPUTE            ──S6
 ABDOMINAL MUSCLE
 THICKNESS
             │
             ▼
   COMPUTE            ──S7
 SUBCUTANEOUS
 FAT AREA
```

```
  COMPUTE VISCERAL   ──S8
    FAT AREA
         │
         ▼
  COMPUTE TOTAL      ──S9
 ABDOMINAL FAT AREA
         │
         ▼
  COMPUTE TRUNCAL    ──S10
  FAT PERCENTAGE
         │
         ▼
  COMPUTE WHOLE      ──S11
  BODY FAT
  PERCENTAGE
         │
         ▼
  COMPARE            ──S12
  SUBCUTANEOUS FAT
  AREA WITH VISCERAL
  FAT AREA
         │
         ▼
  DISPLAY RESULTS    ──S13
         │
         ▼
      ┌──S14
  MEASUREMENT         YES
 SWITCH PRESSED? ────────
         │
         NO
         │
         ▼
       END
    (POWER OFF)
```

## FIG. 4

SUBCUTANEOUS FAT THICKNESS COMPUTED
BY COMPUTING EQUATION (cm)

## FIG. 5

ABDOMINAL MUSCLE THICKNESS COMPUTED
BY COMPUTING EQUATION (cm)

## FIG. 6

## FIG. 7

## FIG. 8

## FIG. 9

## FIG. 10

## FIG. 11

# FIG. 12A

PRESS ELECTRODES AGAINST
FRONT SIDE OF ABDOMEN,
FIT WIDTH MEASURING PARTS
TO SIDES, AND PRESS
MEASUREMENT SWITCH.

# FIG. 12B

## VISCERAL FAT TYPE !

| | |
|---|---|
| SUBCUTANEOUS FAT THICKNESS | 0.5cm |
| ABDOMINAL MUSCLE THICKNESS | 1.5cm |
| SUBCUTANEOUS FAT AREA | 50cm |
| VISCERAL FAT AREA | 100cm |
| TOTAL ABDOMINAL FAT AREA | 150cm |
| TRUNCAL FAT PERCENTAGE | 20.5% |
| WHOLE BODY FAT PERCENTAGE | 20.3% |

CONDITION OF
YOUR ABDOMEN

# FIG. 12C

## SUBCUTANEOUS FAT TYPE !

| | |
|---|---|
| SUBCUTANEOUS FAT THICKNESS | 3.5cm |
| ABDOMINAL MUSCLE THICKNESS | 1.5cm |
| SUBCUTANEOUS FAT AREA | 100cm |
| VISCERAL FAT AREA | 50cm |
| TOTAL ABDOMINAL FAT AREA | 150cm |
| TRUNCAL FAT PERCENTAGE | 20.5% |
| WHOLE BODY FAT PERCENTAGE | 20.3% |

CONDITION OF
YOUR ABDOMEN

# FIG. 12D

PRESS MEASUREMENT
SWITCH IF YOU WISH TO
MEASURE AGAIN.

FIG. 13A

FIG. 13B

FIG. 13C

# FIG. 14

# FIG. 15A

# FIG. 15B

# FIG. 16A

51

NAVEL
SIDE

LEFT
SIDE

RIGHT
SIDE

BACK
SIDE

# FIG. 16B

51

# FIG. 17A

# FIG. 17B

# FIG. 18A

# FIG. 18B

# FIG. 19A

202

271

276a
275a
276b
275b

277a

277b

272a 273a 274a 277b 274b 273b 272b

NAVEL
SIDE

LEFT
SIDE

RIGHT
SIDE

BACK
SIDE

# FIG. 19B

202

271

272a 273a 274a 274b 273b 272b

**EP 1 621 131 B1**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2001178697 A
- JP 2001252256 A
- JP 2001252257 A
- JP 2002238871 A
- JP 2002369806 A
- JP 2004135698 A
- JP 2004141186 A
- US 2004077969 A
- EP 121898 A